# EUROPEAN PATENT SPECIFICATION

(11) **EP 1 759 683 B1**
(45) Date of publication and mention of the grant of the patent: **12.10.2011**
(21) Application number: 05741309.8
(22) Date of filing: 13.05.2005
(51) Int. Cl.: A61K 8/97, A61K 8/98, A61K 8/99, A61Q 7/00

(54) **SCALP AND HAIR-CARE COMPOSITION**
KOPFHAUT- UND HAARPFLEGEZUSAMMENSETZUNG
COMPOSITION DE SOIN DU CUIR CHEVELU ET DES CHEVEUX

(30) Priority: 21.05.2004 JP 2004152083; 15.02.2005 JP 2005037942
(43) Date of publication of application: 07.03.2007
(73) Proprietor: Furusatomura Development Center Co., Ltd., Oomachi-shi Nagano 398-0002 (JP); Goino, Tadashi, Azumino-shi Nagano 399-8301 (JP)
(72) Inventor: GOINO, Tadashi, Minamiazumi-gun Nagano 3998301 (JP); UMETSU, Noriko, Higashichikuma-gun Nagano 3997701 (JP); KITAMURA, Tsuzuki, 4640833 (JP)
(74) Representative: Kramer - Barske - Schmidtchen
(86) International application number: PCT/JP2005/009219
(87) International publication number: WO 2005/112877

(56) References cited:
- WO-A2-01/56589
- DE-A1- 10 050 669
- DE-A1- 10 324 158
- DE-A1- 19 901 732
- JP-A- 5 345 726
- JP-A- 9 136 839
- JP-A- 11 292 786
- JP-A- 60 222 409
- JP-A- 2003 040 787
- JP-A- 2003 040 795
- JP-A- 2003 048 847
- JP-A- 2003 192 541
- JP-A- 2005 060 320
- MD-F1- 1 877
- US-A1- 2003 021 857
- US-A1- 2003 104 005
- DATABASE EPODOC EUROPEAN PATENT OFFICE, THE HAGUE, NL; 5 October 1994 (1994-10-05), XP002513540 & CN 1 092 976 A (YIN ZHIKUAN [CN]) 5 October 1994 (1994-10-05)
- DATABASE EPODOC EUROPEAN PATENT OFFICE, THE HAGUE, NL; 21 April 2004 (2004-04-21), XP002513541 & CN 1 490 036 A (WANG MINDA [CN]) 21 April 2004 (2004-04-21)

## Description

### Technical field

The present invention relates to a composition for scalp and hair of scalp which can inhibit and prevent hair loss and epilation and promote and maintain hair growth.

### Background Art

Conventionally, in order to prevent alopecia and epilation which is at the pre-stage of alopecia and canities, it is important to routinely keep the scalp clean and supply a suitable hair growth component. In order to keep the scalp clean, usually, it is general to wash the scalp and the hair with a wash containing a surface active agent such as shampoo and the like and remove sebum cutaneum as well as the dirt and subsequently, supply a hair growth formula to the scalp.

However, sebaceous membrane which is a barrier of the scalp is broken by the surface active agent contained in shampoo. On the other hand, in usual hair washing, a surface active agent, a silicon resin component and the like easily remain on the scalp as well as it is difficult to sufficiently remove the dirt of the scalp and pit, stratum corneum and lipoperoxide, the occlusion of the pit and the like are generated by these attaching to the pit and hair, coagulating, dissolving hair root and hair follicle. As a result of these, sebum cutaneum is accumulated within hair follicle on the scalp due to secretory abnormality of sebum cutaneum and the reverse flow of sebum cutaneum, whereby it becomes major causes of epilation, itching, and dandruff. Even if a drug or cosmetics having a hair growth stimulant effect is applied in the state described above, the hair growth cycle and cell cycle are disrupted, the sebum cutaneum secretion is still more inhibited, the sebum cutaneum secretion disorder becomes worse, and atrophy of hair matrix tissue cell and the retention of blood flow of the peripheral vessel are generated, and the scalp loses elasticity and becomes hardened. In addition to these, the hair growth cycle becomes abnormal, the hair loss and epilation are generated by means of the hair root function lowering or stoppage by hardening of the scalp and then, the decrease of the hair begins.

In Japanese Patent Laid-Open No. 2003-40795 Gazette, there have been a disclosure that as for a composition containing water soluble extraction component of fungi belonging to basidiomycetes, a water soluble extraction component of the root of Araliaceae plant and/or an extraction component of water/alcohol mixture, there are a variety of kinds of physiological activity actions such as cholesterol lowering action and the like.

The fats and fatty oils squeezed from the fruits and seeds of the plant of Hippophae rhamnoides L. (Sea buckthorn) of Elaeagnaceae is different from the viewpoint of its composition and property from the conventional vegetable fats and fatty oils and animal fats and fatty oils which have been frequently used. For example, in Japanese Patent Laid-Open No. 2001-163792 Gazette and Japanese Patent Laid-Open No. 2002-34506 Gazette, a composition and food for nutrient tonic health promotion and the like which utilize the fats and fatty oils squeezed from the fruits and seeds of the plant of Hippophae rhamnoides L (Sea buckthorn) of Elaeagnacease are known. Up to now, it has not been considered that such fats and fatty oils are used so that these are separated into water content and fats and fatty oils, dispersed and stabilized or promoted to be dispersed to be contained in the composition such as food, cosmetics, pharmaceuticals and the like.

US 2003/021857 A1 relates to a composition containing an extract from Wisteria floribunda, Myristica fragrans, Punica granatum, Trapa bispinosa or a derivative thereof, Coix lacryma-jobi, Elfvingia applanata and Ganoderma lucidum or a derivative thereof, wherein the composition further contains Panax ginseng or Panax japonicus (Araliaceae) extracts. JP 09-136839 A discloses a composition containing an extract of Wisteria floribunda, Terminalia chebula, Trapa japonica, Coix lacryma-jobi, Elfvingia applanata and Ganoderma lucidum, wherein the composition preferably contains Panax ginseng C.A. Meyer.

US 2003/104005 A1 relates to a composition comprising extracted components of at least one type of Basidiomycotina that belongs to polyporaceae Basidiomycotina and extracted components of the root of a plant that belongs to Araliaceae.

JP 60-222409 A discloses a hair tonic composition containing a ginseng extract (consisting essentially of saponin), a licorice extract (consisting essentially of glycyrrhizin), and a Shiitake mushroom mycelial extract (consisting essentially of amino acids and vitamins).

DATABASE EPODOC EPO, THE HAGUE, NL, XP002513540 relates to a skin care lotion containing 1 to 8 % ginseng, 1 to 8 % glossy ganoderma, 1 to 10 % pollen and 0.5 to 5 % royal jelly.

DATABASE EPODOC EPO, THE HAGUE, NL, XP002513541 relates to a chinese medicine for growing and blackening hair and delaying senility prepared from the oil of seabuckthorn (Hippophae chammoides) fruit and leaf.

DE 100 50 669 A1 discloses a hair growth agent for external use, obtained by boiling Reishi (Ganoderma lucidum), Angelica chinensis, Astralagus, Red ginseng and sea algae in water and then diluting the obtained extract with water and ethanol.

DE 199 01 732 A1 relates to an agent having an anti-tumor effect and containing Reishi fungus (Ganoderma lucidum), Kavaratake fungus (Coriolus versicolor) and ginseng root (Angerica shikokiana makino) in a ratio of 10:5:3.

JP 2003-048847 A discloses a bioactive composition and a method for producing the same, wherein the composition contains Ganoderma lucidum, Coriolus versicolor and a root of a plant belonging to Araliaceae as active ingredients.

JP 2003-040795 A relates to an antihyperlipidaemic composition and a method for manufacturing the same, wherein the composition contains one or more kinds of basidiomycetes and a root of a plant belonging to Araliaceae as active ingredients.

MD1877F (abstract) discloses a hair growth stimulator containing bee honey, extract of camomile flowers and oil, and in addition badger grease, matured wine distillate, ethyl alcohol, iodine alcoholic solution, onion and garlic extract, and jojoba and almond oil, wherein the amount of bee honey in the hair growth stimulator is 0.3 to 0.6 mass %.

JP 2003-192541 A discloses a skin care preparation containing a hair growth-promoting agent, containing royal jelly or a glycoprotein obtained from royal jelly having a molecular weight of 57 kDa, wherein the royal jelly has a vascular endothelial growth factor production-promoting activity, a production promoting activity, an endothelial cell proliferation-promoting activity and a lumen formation promoting activity.

### Disclosure of the Invention

An object of the present invention is to provide a composition for scalp and hair of scalp which realizes hair growing, hair growth or the like by arranging the scalp environment without inhibiting the function of sebaceous membrane and dark cell (superficial type cell) of scalp. Moreover, another object of the present invention is to provide a composition for scalp and hair of scalp containing fats and fatty oils which is capable of effectively maintaining a hair growing and hair growth components in the case where these components are contained as well as to appropriately refill the sebaceous membrane of scalp.

The present inventors have found that the composition combined between the extraction component of basidiomycetes and the extraction component of the root of the plant of Araliaceae is excellent in protection of hair and hair growing as well as it makes the sebaceous membrane and cells of scalp normally function. Moreover, the present inventors have found that the composition which is excellent in protection of hair as well as appropriately supplements fat and fatty oil contents to the scalp and hair, keeps a hair growth component and the like in an effective dispersed state, and makes the sebaceous membrane and cells normally functions without chemically damaging these can be obtained by using the fats and fatty oils squeezed from the fruits and seeds of the plant of Hippophae rhamnoides L. (Sea buckthorn) of Elaeagnaceae as fat and fatty oil contents of the composition for washing the scalp and hair. According to these acknowledgments, the following means are provided.

The present invention is directed to a composition for scalp and hair of scalp, the composition containing extraction component of fungi belonging to basidiomycetes and extraction component of the root of a plant of Araliaceae. The fungi belonging to the basidiomycetes preferably contain fungi belonging to Aphyllophorales Ganodermataceae Ganoderma lucidum and fungi belonging to Aphyllophorales Polyporaceae Coriolus. The fungus belonging to the Ganoderma lucidum is preferably Reishi Fungus. The fungus belonging to the Coriolus is preferably Coriolus Versicolor. The plant of Araliaceae is preferably Panax japonicus C. A. Meyer.

Further in the composition, the extraction component of fungus belonging to the basidiomycetes and extraction component of the root of the plant of Araliaceae are preferably water extraction component and/or water/alcohol mixture extraction component, respectively. Especially, the water extraction component of fungus belonging to the basidiomycetes preferably contains the component in the extraction liquid obtained by immersing the cultured matter of fungus belonging to the basidiomycetes, by blocking the light and by still standing and preserving it in the range from 5°C to 40°C. The root of the Araliaceae preferably contains the component in the extraction liquid obtained by immersing the root of the Araliaceae, by blocking the light and by still standing and preserving it in the range from 5°C to 40°C.

Further, it is preferable that the composition has raw material composition in the range from 0.2 portions by weight to 20 portions by weight of the root of a plant of the Araliaceae with respect to 20 portions by weight of fungi belonging to the basidiomycetes. According to the present invention, the composition further contains honey and/or royal jelly. The composition further contains squid ink component preferably. According to the present invention, the composition further contains fats and fatty oils of Hippophae rhamnoides L. whose raw material is fruit and/or seed of Elaeagnaceae Hippophae rhamnoides L. plant preferably.
Fig. 1 is a photograph showing the alopecia site before the test period of the subject 2 in Example 1;
Fig. 2 is a photograph showing the alopecia site in Fig. 1 at the time when the test period of the subject 2 in Example 1 was completed;
Fig. 3 is a photograph showing the alopecia site before the test period of the subject 3 in Example 1; and
Fig. 4 is a photograph showing the alopecia site in Fig. 3 at the time when the test period of the subject 3 in Example 1 was completed.

A composition for hair of scalp of the present invention contains the extraction component of fungi belonging to basidiomycetes and the extraction component of the root of Araliaceae plant. The action to the scalp and hair in the composition of the present invention has exceeded over the prediction of those skilled in the art and the present inventors. It is known that this composition has the physiological activity such as an anti-tumor activity and the like. However, the action at the time when this composition has been applied to scalp or hair of scalp as an external preparation is not known before the present application has been filed, and the improvement of scalp environment at the time when it is applied to the local site such as the scalp and the like and the actions of hair growing, the hair growth and the like is usually not grasped as an action relating to an anti-tumor activity and the like which have been already disclosed.

Moreover, the composition for scalp and hair of scalp of the present invention contains Hippophae rhamnoides L. (Sea buckthorn) fats and fatty oils. In a composition of the present invention, Hippophae rhamnoides L. (Seabucktorn) fats and fatty oils is a fat and fatty oil which can appropriately supply the sebaceous membrane of hair scalp by itself and the infiltration of these components to the scalp and the like can be secured by effectively dispersing and maintaining these components at the time when it contains hair growing and hair growth component and the like as well.

Hereinafter, concerning with Embodiments of the present invention, a composition for scalp and hair of scalp of the present invention and preferable applied modes of the present composition for scalp and hair of scalp will be explained and a method of manufacturing a composition for scalp and hair of scalp of the present invention will be explained.

### (Composition for scalp and hair of scalp)

### (Hippophae ramnoides L. fat and fatty oil)

### (Elaeagnaceae Hippophae rhamnoides L. plant)

Fats and fatty oils of Hippophae rhamnoides L. are fats and fatty oils obtained from the fruits and/or seeds of Elaeagnaceae Hippophae rhamnoides L. plant as the raw material. As Elaeagnaceae Hippophae rhamnoides L. plant, H. salicifolia, H. thibetana, H. neurocorpa, H. rhamnoides and the like are listed. Any one of these can be used. Most generally speaking, H. rhamnoides. These may be singly used or two kinds or more may be used in combination.

As for this plant, any of the fruits (pulp, pericarp), seed, stem, leaf and root can be used as the raw material. It is preferable that the portion containing the fruits or seed is used as the raw material. It is more preferable that only the fruits and seed are used as the raw material. The form of the raw material is not particularly limited, the plant body or its one portion as it is, workings which has been dried, finely cut, broken and so on can be also used.

As a method of obtaining fats and fatty oils from the above-described plant body raw material, these can be obtained by utilizing a method of pressing and squeezing the raw material or a method of extracting using a non-polar solvent such as hexane and the like as an extraction solvent. In the case where a method of pressing and squeezing is used, it is preferable that the raw material is to be broken. Moreover, in the case where a method of solvent extraction is used, as an extraction solvent, the solvent such as hexane and the like which can be used for collecting fats and fatty oils from the plant body raw material can be generally used. Moreover, the extraction can be performed by adding the solvent having 2 to 10-fold weight with respect to the plant body raw material and if it is necessary, by heating and immersing and extracting for a suitable time period. Subsequently, unsolved matter is removed by filtering and the like if it is necessary, and furthermore, the fats and fatty oils can be obtained by distilling away the solvent. It should be noted that as for squeezing oil from the plant body raw material, the conventional known variety of kinds of methods can be applied by appropriately changing these and so on. Moreover, supercritical extraction can be also used.

Referring to the amount of fats and fatty oils of Hippophae rhamnoides L. in the present composition, although it depends upon its intended use, 0.5% by weight or more is preferable. Because if it is 0.5% by weight or more, it can exert the effect of refilling sebaceous membrane. Moreover, 70% by weight or less is preferable. In the case where it exceeds over 70% by weight, fats and fatty oils of Hippophae rhamnoides L. becomes in a turbid state or separated. Moreover, it depends upon its intended use, 10% by weight or more is preferable.

### (Honey and Royal Jelly)

The present composition contains honey and/or royal jelly. Honey and royal jelly can enhance the finishing work, the smoothness of combing by maintaining the moisture of scalp and hair of scalp after the present composition has been applied to the scalp and hair of scalp. Moreover, royal jelly has still further the significant effect of maintaining the moisture to the scalp and hair of scalp. It is preferable that both of honey and royal jelly are used. It is more preferable that these are blended in a ratio range from 3 portions by weight of royal jelly with respect to 1 portion by weight of honey to 10 portions by weight of royal jelly with respect to 1 portion by weight of honey. In the case where it is in the above-described range, the uniform mixture state can be easily obtained as well as the functions of both of honey and royal jelly can effectively exert. It is more preferable that these are blended in a ratio range from 5 portions by weight of royal jelly with respect to 1 portion by weight of honey to 8 portions by weight of royal jelly with respect to 1 portion by weight of honey. It is preferable that honey and royal jelly have been previously mixed. When fats and fatty oils of Hippophae rhamnoides L. are mixed with the above-described mixture, these are easily mixed with fats and fatty oils of Hippophae rhamnoides L. to be uniformed, and the separation after the mixture can be suppressed. It should be noted that it is preferable that honey and royal jelly are components contained in the present composition also from the viewpoints that the peculiar flavor of fats and fatty oils of Hippophae rhamnoides L. can be suppressed as well as honey and royal jelly are well dispersed.

As for honey and royal jelly, if it is possible to be usually available, it can be used without any limitation of its source. According to the present invention, as the total amount, honey and/or royal jelly are contained in the composition in the range from 3 portions by weight to 15 portions by weight with respect to 1 portion by weight of fats and fatty oils of Hippophae rhamnoides L. Because if these are contained in the above-described range, the peculiar flavor of fats and fatty oils of Hippophae rhamnoides L. can be suppressed as well as honey and/or royal jelly are well dispersed by means of fats and fatty oils of Hippophae rhamnoides L. Moreover, in the case where it is less than 3 portions by weight, it becomes easily in an ununiform mixture state, and in the case where it exceeds over 15 portions by weight, it tends to show the ununiform dispersion, the separation in a liquid state and the nature of precipitation. More preferably, it is 10 portions by weight or less.

According to the present invention the total amount of honey and/or royal jelly in the present composition is in the range from 5% by weight to 90% by weight. In the case where it is in the above-described range, as a composition, an excellent dispersion or uniform state can be easily obtained. Preferably, it is 80% by weight or less.

### (Extraction component of basidiomycetes and extraction component of root of Araliaceae plant)

### (Basidiomycetes)

The extraction component of fungi belonging to Basidiomycetes is contained in the present composition. As a basidiomycetes, it is not particularly limited, however, it is preferable that fungi belonging to Aphyllophorales is used, and it is more preferable that fungi belonging to Ganodermataceae and/or Polyporaceae are used.

As a fungus belonging to Ganodermataceae, Ganoderma Lucidum, especially, Reishi Fungus can be exemplified. This fungus originally breeds on the trees by preference, however, the native fungi are scarce. It should be noted that the artificial culture could be carried out. This fungi has a glossy cap portion in a wax state and axial portion, the length of the axis reaches to about 15 cm. The color of the fruit body shows red color, yellow color, white color, violet color and black color. This fungus grows on the stump or nearby the base of the tree which becomes weak by a certain disease and becomes a white filamentous body.

Moreover, as a fungus belonging to Polyporaceae, a fungus of Coriolus, especially, Coriolus Versicolor can be exemplified. This fungus grows in nature in the west areas of Japan, especially, in Shinshu District (espeically, Nagano Preferecture), Shikoku District, and Kyushu District. This fungus is originally xylophile fungus, and especially, fond of broad leaf trees.

In the present composition, the extraction component (extraction component of water and/or water/alcohol mixture) obtained by utilizing one or two species or more of these fruit bodies of fungi, either of fungus mycelium and cultured matter (culture solution and the like) as a raw material can be contained as an effective component. Both of fungi belonging to Ganodermataceae and Coriolus may be used, only any one of these may be used. It is preferable that Ganodermataceae and Coriolus may be used. It is more preferable that Reishi Fungus and Coriolus Versicolor are used, and it is more preferable that only Reishi Fungus and Coriolus Versicoloro are used.

It should be noted that basidiomycetes might be a fungus growing in nature or a fungus might be grown by artificial means or by a cell culture, which is not particularly limited. It is preferable that a fungus growing in nature is used. The form of these fungi which are used in the present invention may be either of the fruit body, fungus mycelium or cultured matter, however, it is preferable that the fruit body is used. Moreover, as for a fruit body, it is preferable that the fruit body which has been air dried while avoiding the light at room temperature is used. Particularly as for a fungus of Ganodermataceae, it is preferable that a black fruit body which has grown and has been matured in nature is used. Moreover, as for a fungus of Coriolus, it is preferable that the fruit body which has grown in nature and has been collected in summer is used, and it is more preferable that the fruit body which has been air dried while avoiding the light at room temperature is used. It should be noted that the fruit body is available as an alcohol immersion liquid, a paste or an extract besides a solid matter which has been dried.

### (Root of Araliaceae plant)

Moreover, in a composition of the present invention, the extraction component of the root of a plant of Araliaceae is contained. As a plant of Araliaceae, it is typically a Panax ginseng. In a medicinal ginseng, besides Panax ginseng C. A. Meyer, P. quinquefolium L., P. notoginseng, P. japonicus C. A. Meyer and the like are contained. As a plant of Araliaceae, one species may be singly used, or two species or more may be used in combination. In the present invention, it is preferable that Panax ginseng C. A. Meyer and/or P. japonicus C. A. Meyer are used. It is particularly preferable that P. japonicus C. A. Meyer is used, and it is the the most preferable that only P. japonicus C. A. Meyer is used. As for these ginsengs, in any case, the root is used.

The root of a plant of Araliaceae such as P. japonicus C. A. Meyer and the like is usually available in a dried state. Or, it is available as an alcohol immersion liquid, or a paste, and an extract.

The root of a plant of Araliaceae may be a root of what is called plant body, however, it may be also a cultured cell. In the case where it is a cultured cell, it is preferable that a cultured cell derived from the root is used. It should be noted that if it contains the raw material derived from the root of a plant of Araliaceae, the raw material containing the other sites than the root of the plant body could be also used.

### (Method of manufacturing extraction component)

The respective extraction component of basidiomycetes and the root of a plant of Araliaceae can be obtained by extracting it using these raw material, and using water, hot water, a lower alcohol such as methanol, ethanol, isopropanol, and n-butanol, or multivalent alcohol such as propylene glycol and 1, 3-butylene glycol, or a mixture between these organic solvent and water, and the like as an extraction solvent. As for an extraction solvent, it is preferable that the extraction is carried out using water (hot water) or a mixture of water/alcohol. It should be noted that it is preferable that the raw material component in these extraction components used for the present composition is selected based on the combinations already described.

The extraction by these various kinds of extraction solvents can be performed according to the conventional method of extracting a plant component. The respective extracted matters may be mixed by extracting the extraction component of basidiomycetes and the extraction component of Araliaceae, per each raw material, respectively, or by extracting two species or more raw materials in combination, or the raw material of basidiomycetes and the root of a plant of Araliaceae may be extracted using a batch processing. It is preferable that after the whole raw materials of the extraction components which are to be contained in the composition has been mixed, it is extracted using hot water. As for the respective raw materials, at the time when the extraction is carried out, it is preferable that it is broken and made it into a spline state or in a powder state, and it is more preferable that it is broken into fine pieces. It is particularly preferable that it is broken into fine pieces in a size of square having the side of about 5 mm or less.

Referring to the blending ratio between basidiomycetes and the root of a plant of Araliaceae in the extraction raw material, it is not particularly limited, however, it is in the ratio range from 0.2 portions by weight of the root of a plant of Araliaceae to 20 portions by weight of the root of a plant of Araliaceae with respect to 20 portions by weight of Basidiomycetes. It is more preferable that it is in the range of ratio from 8 portions by weight of the root of a plant of Araliaceae to 12 portions by weight of the root of a plant of Araliaceae with respect to 20 portions by weight of basidiomycetes. Moreover, it can be also set so that it is in the ratio range from 2 portions by weight of the root of a plant of Araliaceae to 4 portions by weight of the root of a plant of Araliaceae with respect to 15 portions by weight of Basidiomycetes. As a fungus of basidiomycetes, in the case where fungi of Ganodermataceae and Coriolus are used, the blending ratio is neither particularly limited. The equivalent weight of these can be also used, respectively, or it can be set so that one portion by weight of fungi of Coriolus is blended with 2 portions by weight of fungi of Ganodermataceae. It should be noted that any one of the ratios of the above-described extraction raw materials is the ratio in dried matter conversion. Hereinafter, particularly, water extraction and water/alcohol mixed liquid extraction will be explained in detail.

### (Water extraction)

Referring to an extraction, it may be extracted with water at normal temperature, or it may be extracted with hot water. For example, a water extraction liquid can be obtained by preserving the raw material in the range from 5°C to 40°C, preferably from 10°C to 37°C, more preferably from 15°C to 25°C in a light blocking state in which the raw material has been immersed in water (preferably, stood still as it is). In the above-described extraction, it is preferable that it is extracted for a long term (from about 2 weeks to about one month, preferably 3 months or more and more preferably one year or more). In the case of the above-described immersion extraction, it is preferable that it is performed in a sealed container. Moreover, although the amount of water with respect to the extraction raw material is not particularly limited, it is preferable that the raw material in the range from 10 portions by weight to 30 portions by weight is extracted using water in the range from about 100 portions by weight to about 1000 portions by weight.

Referring to the temperature of hot water at the time when the extraction is carried out using hot water, it is set so that it is in the range from 80°C to 100°C, preferably from 90°C to 95°C. Moreover, referring to the extraction time, it is preferable that it is extracted for at least one hour, preferably 2 hours or more, and further preferably 2 and a half hours or more. Moreover, it is set so that the upper limit becomes in the range from 3 hours to 4 hours. It is preferable that the extraction operation is performed using a reflux condenser. It should be noted that the weight of water with respect to the raw material is not particularly limited, however, it is preferable that it is extracted using water in the range from about 100 portions by weight to about 1000 portions by weight with respect to the raw material which is in the range from 10 portions by weight to 30 portions by weight.

As one example of water extraction, the following example will be listed: in order to manufacture the present composition from the extraction raw materials at the blending ratio (weight ratio) of about 10 : about 5 : about 3 of the fruit body of Ganoderma lucidum : the fruit body of Coriolus versicolor : the root of P. japonicus C. A. Meyer which are the preferable raw material composition in the present invention, for example, 10 g of the fruit body of Ganoderma lucidum, 5 g of the fruit body of Coriolus versicolor and 3 g of P. japonicus C. A. Meyer are collected respectively, mixed, broken into the fine pieces having the size of a square having the side of about 5mm, and distilled water in the range from 500 mL to 1000 mL is added to this broken matter, it is boiled for 3 hours using a reflux condenser, and after it has been filtered, it is made composition (stock solution) for use in the present invention.

It should be noted that it is preferable that in the hot water extraction operation, a container of the atmospheric air opening type, particularly, the material in which the metal portion has been coated with a corrosion resistant coat or worked and the like is used besides a container made of glass, a container made of porcelain enamel, a container made of ceramic and the like. Moreover, it is preferable that it is carried out by utilizing a reflux condenser. As for the heating extraction operation, it is not particularly limited, however, in the case where the above-described raw material versus extraction liquid weight is used, it is made so that the extraction liquid becomes about 60% of the initial time. In the case where the extraction water weight which has been initially added to the above-described extraction raw material is 800 mL, it is preferable that it is carried out so that the extraction liquid weight becomes about 500 mL.

### (Water/alcohol mixed liquid extraction component)

In the case where it is extracted using water/alcohol mixed liquid, as an alcohol which is to be used, it is preferable that ethanol is used. In the case where it is extracted with water/alcohol mixed liquid, it is preferable that its alcohol concentration is equal to 50 v/v% or less, and it is more preferable that it is in the range from about 30 to about 40 v/v%. It is the most preferable that it is about 35 v/v%. The extraction liquid containing alcohol can be also obtained by immersing the raw material into water/alcohol mixed liquid and preserving it at normal temperature (preferably, stood still as it is) and so on. It is preserved in the range from 5°C to 40°C in a light blocking state, preferably from 10°C to 37°C, and more preferably from 15°C to 25°C. In this case, as for the preservation period, it can be preserved in the range of a long period from about two weeks to about one month, preferably for 3 months or more, and more preferably for one year or more. Moreover, it is preferable that it is carried out in a sealed container. Moreover, the mixed liquid weight used with respect to the extraction raw material is not particularly limited, however, it is preferable that the mixture liquid is made in the range from 100 portions by weight to 1000 portions by weight with respect to the raw material in the range from 10 portions by weight to 300 portions by weight.

It should be noted that the heating extraction can be also carried out. In the case where it is heated, it is preferable that it is in the range from 50 to 80°C, it is more preferable that it is 75°C or less, and it is still more preferable that it is 70°C or less. Although the heating extraction time is not particularly limited, it is preferable that it is 10 hours or more, it is more preferable that it is 20 hours or more, and it is the most preferable that it is about 30 hours or more. It should be noted that in order to easily regulate the component of the composition after that, it is preferable that the concentration of alcohol is 20v/v% or less.

Therefore, in the case where it is extracted with alcohol solution at a concentration exceeding over 20v/v%, it is preferable that it is made so that it is 20v/v% by diluting it with water after the extraction. Moreover, also in the case where it is heated and extracted, the mixed liquid weight used with respect to the extraction raw material is particularly limited, however, it is preferable that the mixed liquid is made in the range from 100 portions by weight to 1000 portions by weight with respect to the raw material in the range from 10 portions by weight to 300 portions by weight. As for a container for extraction, it is preferable that a container similar to that used in the case where it is extracted with hot water is used. In the extraction operation, it is preferable that it is carried out by utilizing a reflux condenser.

As a typical example in the case where fungi of Ganodermataceae and fungi of Coriolus which are basidiomycetes are extracted with water/alcohol mixed liquid, the following operations can be listed below: it is made 1000 mL by adding 35v/v% ethanol solution to 150 g of Ganoderma lucidum broken into fine pieces (preferably, fruit body, preferably, in a spline state), and this liquid is decocted while the temperature is maintained at about 70°C for 30 hours. Or, it is immersed for one month at room temperature in a dark room. After 30 hours has passed at 70°C, or one month has passed at room temperature, hot water (or water) can be added so that the whole weight of it becomes 1000 mL. It should be noted that it is preferable that the alcohol concentration becomes finally about 20v/v%.

Moreover, as a typical example in the case where the root of a plant of Araliaceae is extracted with water/alcohol mixed liquid, the following operations can be listed: it is made 1000 mL by adding 35v/v% ethanol solution to 30 g of the dried root of a plant of Araliacae which has broken into fine pieces, and this liquid is decocted while the temperature is maintained at about 70°C for 30 hours. Or, it is immersed for one month at room temperature in a dark room. After 30 hours has passed at 70°C, or one month has passed at room temperature, hot water (or water) can be added so that the whole amount of it becomes 1000 mL.

As these extraction liquids, with respect to Basidiomycetes and the root of a plant of Araliaceae, respectively, both or either of water extraction liquid and water/alcohol mixed extraction liquid can be used.

Moreover, the raw material of basidiomycetes and the root raw material of a plant of Araliaceae can be also singly extracted, however, the raw material composition containing both can be also extracted with the same extraction medium, or both can be extracted at the same time with the same extraction medium. Also in the case where both are extracted at the same time the extraction conditions in the above-described raw material of Basidiomycetes or the root raw material of a plant of Araliaceae can be employed.

Hereinafter, a typical example of the extraction performed at the same time will be shown below. Decoction is performed in the above-described heating range by adding 800 mL of hot water with respect to 10 - 15 g of fruit body of Ganoderma lucidum, 3 g of the root of Panax japonicus C. A. Meyer which has been broken into a square having the side of 5 mm or less, preferably in the range from about 0.5 to about 2 mm in a spline state so that the remaining amount of the extraction liquid becomes 500 mL. It is preferable that it is heated and regulated for about 30 minutes so that it becomes 500 mL. Or, it is immersed for one month at room temperature in a dark room. After 30 hours have passed at 70°C or one month has passed at room temperature, the whole amount is regulated if it is necessary. Moreover, it is made 1000 mL by adding 35 - 40v/v% ethanol solution to the raw material mixture having the similar form and composition (however, 100 - 150 g of the fruit body of Ganederma lucidum, 30 g of the root of Panax japonicus C.A.Meyer), and this liquid is decocted for about 30 hours while maintaining this liquid at about 70°C. Or, it is immersed for one month at room temperature in a dark room. After 30 hours have passed at 70°C or one month has passed at room temperature, hot water (or water) is added so that the whole amount becomes 1000 mL. It should be noted that it is preferable that the alcohol concentration finally becomes about 20 v/v%.

As for the obtained extraction liquid, the extraction remaining portion may be removed by performing the filtration and the like if it is necessary. It is preferable that as for the extraction liquid prepared in this way, the oxidation-reduction potential is 1230 mV or less at the time when it is dissolved. It is more preferable that the extraction liquid whose oxidation-reduction potential is 900 mV or less is used as a hair growing and hair growth component. It is preferable that the oxidation-reduction potential is a value measured in an aqueous solvent, preferably in water. As for the stock solution obtained by the above-described extraction, or its aqueous diluted solution, its oxidation-reduction potential can be measured as it is. In the case where the extraction liquid has been solidified, it is measured in a state where it is dissolved, suspended in a suitable solvent, water or the like.

It is further preferable that the oxidation-reduction potential of the present extraction liquid is 330 mV or less, more preferably 300 mV or less, and still more preferably 250mV or less. It is the most preferable that it is 0 mV or less. Moreover, it is preferable that the oxidation-reduction potential is -1200mV or more, and it is more preferable that it is -300mV or more.

Moreover, it is preferable that the pH of the solution of the present extraction liquid is in the range from 4.5 to 6.5. It is because the weak acidic area is an area in which it is suitable for a living cell, and the active component of the present extraction liquid most effectively functions.

The extraction liquid obtained in this way, for example, water extraction liquid and/or water/alcohol mixture extraction liquid can be made as an active component of the present composition as it is, or it can be also used as an active component of the present invention by suitably diluting it. Moreover, the dried extraction component is obtained by further drying it. It should be noted that by considering the form of the present composition, the additive agent for preparing the pharmaceutical or stabilizing the extraction component could be also added at the time when the extraction liquid is concentrated or dried. Particularly, for dosage form in a liquid form, the extraction liquid is easily used as it is, or by concentrating or diluting it if it is necessary.

In the present composition, as for the content of the various kinds of extraction components, it is not particularly limited, however, it is preferable that it is 0.05% by weight or more as the dried weight of the extraction component, it is more preferable that it is 0.1% by weight or more, and it is still more preferable that it is 0.5% by weight or more. Moreover, it is preferable that it is 5% by weight or less. It should be noted that these extraction components might be artificially manufactured based on the extraction component and its composition.

### (Other components)

In a composition of the present invention, the other effective components to be contained are not prevented. In an area where the actions of the extraction component, Hippophae rhamnoides L. fats and fatty oils and the other components in a composition of the present invention, the other components can be contained. As the above-described other components, usually, the other components used for cosmetics, a pharmaceutical and a quasi drug, for example, besides various kinds of active components for scalp and hair of scalp, various kinds of oily or aqueous components, a surface active agent , a moisturizing agent, a thickening agent, an antiseptic agent, a ultraviolet-ray absorbing agent, an anti-oxidizing agent, a perfume, a coloring agent, water/alcohol, a variety of species of drugs and the like are listed.

It is preferable that the alcohol concentration in the present composition is made 10% by weight or less of the whole composition. Because if it is 10% by weight or less, the stimulating property to scalp is suppressed. It is more preferable that it is 5% by weight or less. In order to regulate the alcohol concentration, the regulation can be performed by usage amount of the above-described water/alcohol mixture extraction liquid and water extraction liquid, and the regulation can be performed by the usage amount of water and alcohol as well.

The pH of the present component is not particularly limited, however, when the influence upon the scalp and hair of scalp is considered in the case where it is used as a usual external preparation, it is preferable that pH of it is in the range from 4.5 to 6.5. When the pH is in this range, hypoallergenic property as well as appropriate permeability to the sebum cutaneum membrane of the scalp and hair of scalp can be secured. It is more preferable that it is in the range from 4.5 to 5.5. Moreover, the oxidation-reduction potential of the present composition is not particularly limited, however, it is preferable that it is in the range from 90 mV to 380 mV. If it is in the range, the protection action of an excellent skin barrier can be exerted.

The present composition can be an external preparation applied to the scalp or hair of scalp as cosmetics, a pharmaceutical and a quasi drug. In the case, the dosage form is optional. For example, if it is a dosage form that is capable of being applied to the external preparation such as a liquid state, an emulsified liquid, an ointment, a cream, a gel, an aerozol and the like, any one of the above-described ones may be available. For example, as a concrete mode suitable for the present composition, for example, a hair tonic, a hair cream, a hair location, a hair spray, a mousse, a shampoo, a rinse, a treatment, a scalp treatment and the like can be listed. Particularly, it is preferable that it is made into a hair lotion, a hair tonic, a shampoo, a coloring agent for hair of scalp and the like.

In order to enhance the fixing property on the scalp and the hair of scalp of the above-described extraction component in the present composition, gum arabic and camellia oil can be added. These can be added in the range from 0.1% by weight to 15% by weight of the whole composition. It is particularly preferable that the above-described blending is used for a lotion, a tonic and the like. Moreover, the hair dressing effect can be also enhanced using glycerin and the like.

Next, the case where the present composition is used as a scalp and hair of scalp washing agent composition and a hair coloring agent composition will be explained below.

According to one Embodiment of the present composition, a composition for washing a scalp and hair of scalp such as a shampoo and the like is provided. In this case, it is preferable that the present composition contains fats and fatty oils of Hippophae rhamnoides L 0.1% by weight or more. If it is 0.1% by weight or more, as a composition for washing a scalp and hair of scalp, the moisture content and fat and fatty oil content can be sufficiently dispersed and the fat and fatty oil content can be refilled with the scalp and hair of scalp as well. It is preferable that it is 0.5% by weight or more, and it is more preferable that it is 1.0% by weight or more. It should be noted that in a composition for washing, it is preferable that it is made so that the upper limit is 10% by weight or less because if it is 10% by weight or less, the washing performance as a composition for washing a scalp and hair of scalp is easily secured.

In a composition for washing a scalp and hair of scalp (shampoo composition), it is preferable that the total weight of honey and royal jelly is 30% by weight or less. Because if it is 30% by weight or less, the washing performance as a composition for washing a scalp and hair of scalp can be secured.

As a preferred example of a shampoo composition, when the foaming and the like as a shampoo is considered, it can be made so that the total weight of water/alcohol mixture extraction liquid and/or water extraction liquid is in the range from 10% by weight to 80% by weight. It is more preferable that it is equal to 15% by weight or more. Moreover, it is more preferable that the upper limit is 60% by weight or less, it is further more preferable that it is 40% by weight or less, and it is still more preferable that it is 35% by weight or less. When the washing property and the like are considered, it is preferable that pH of it is in the range from 6.0 to 8.5.

To enhance the usability and texture as a shampoo and suppress the friction of hair in washing, a surface active agent such as a metal salt of fatty acid generally used in a composition for washing the scalp and hair of scalp. As the above-described surface active agent, it is not particularly limited, however, for example, in the case where it is an anionic surface active agent, fatty acid soap (for example, sodium laurate, sodium palmitate, and the like); higher alkylsulfuric ester salt (for example, sodium lauryl sulfate, potassium lauryl sulfate and the like); alkylethersulfuric ester salt (for example, POE-triethanol amine lauryl sulfate, POE-sodium lauryl sulfate and the like); N-acylsarcosinic acid (for example, lauroyl sarcosine sodium and the like); higher fatty acid amide sufonate (for example, N-myristoyl - N - methyl taurine sodium, coconut oil fatty acid methyl taurine sodium, laurylmethyl taurine sodium and the like); phosphoric ester salt (POE- sodium oleyletherphosphate, POE-stearylether phosphoric acid and the like); sulfosuccinate (for example, di-2-ethylhexyl sodium sulfosuccinate, monolauroyl monoethanol amide polyoxyethylene sodium sulfosuccinate, laurylpolypropylene glycol sodium sulfosuccinate and the like); alkylbenzene sulfonate (for example, linear dodecylbenzene sodium sulfonate, linear dodecyl benzene triethanol amine sulfonate, linear dodecyl benzene sulfonic acid and the like); higher fatty acid ester sulforic ester salt (for example, cured coconut oil fatty acid glycerin sodium sulfonate and the like); N-acylglutamate (for example, N-lauroyl monosodium glutamate, N-stearoyl disodium glutamate, N- myristoroyl - L - monosodium glutamate and the like); sulfated oil (for example, sulfonated castor oil and the like); POE - alkyl ether carboxylic acid, POE - alkylallyl ether carboxylate, alpha - olefin sulfonate, higher fatty acid ester sulfonate; secondary alcohol sulfuric ester salt; higher fatty acid alkylol amid sulfuric ester salt; lauroyl monoethanol amide sodium succinate; N- ditriethanol palmitoyl aspartate; casein sodium and the like are listed.

As a amphoteric surface active agent, for example, imidazolin based amphoteric surface active agent (for example, 2-undecil -N, N, N-(hydroxyethyl carboxymethyl)-2-imidazoline sodium, 2-ccocoyl-2-imidazolinium hydroxide-1 carboxyethyloxy-2-sodium salt and the like); betaine based surface active agent (for example, 2-heptadecyl-N-carboxymethyl-N-hydroxyethyl imidazolinium betaine, lauryl dimethyl aminoacetic acid betaine, alkylbetaine, amide betaine, sulfobetaine and the like) and the like are listed.

As a lipophilic nonionic surface active agent, for example, sorbitan fatty acid esters (sorbitan monooleate, sorbitan monoisostearate, sorbitan monolaurate, sorbitan monopalmitate, sorbitan monostearate, sorbitan sesquioleate, sorbitan trioleate, penta-2-ethylhexylic acid diglycerol sorbitan, tetra-2-ethylhexylic acid diglycerol sorbitan and the like); glycelin polyglycerin fatty acids (for example, mono-cottonseed oil fatty acid glycerin, monoerucic acid glycerin, sesquioleic acid glycerin, monostearic acid glycerin, alpha, alpha'-oleinic acid pyroglutamic acid glycerin, monostearic acid glycerin malic acid and the like) ; propylene glycol fatty acid esters (for example, propylene glycol monostearate and the like); cured castor oil derivative; glycerin alkyl ether and the like are listed.

As a hydrophilic nonionic surface active agent, for example POE-sorbitan fatty acid esters (for example, POE-sorbitan monooleate, POE-sorbitan monostearate, POE-sorbitan monooleate, POE-sorbitan tetraoleate and the like); POE sorbit fatty acid esters (for example, POE-sorbit monolaurate, POE-sorbit monooleate, POE-sorbit pentaoleate, POE-sorbit monostearate and the like); POE-glycerin fatty acid esters (for example, POE-monooleate such as POE-glycerin monostearate, POE-glycerin monoisostearate POE-glycerin triisostearate and the like); POE-fatty acid esters (for example, POE-distearate, POE-monodioleate, ethylene glycol distearate and the like); POE-alkyl ethers (for example, POE-lauryl ether, POE-oleyl ether, POE-stearyl ether, POE-behenyl ether, POE-2-octyl dodecyl ether, POE-cholestanol ether and the like); pluronic types (for example pluronic and the like); POE-POP-alkyl ethers (for example, POE-POP-cetyl ether, POE-POP-2-decyltetradecyl ether, POE-POP-monobutyl ether, POE-POP-hydrogenated lanolin, POE-POP-glycerin ether and the like); tetra POE-tetra POP-ethylene diamine condensed matters (for example, tetronic and the like); POE-castor oil cured castor oil derivative (for example, POE-castor oil, POE-cured castor oil, POE-cured castor oil monoisostearate, POE-cured castor oil triisostearate, POE-cured castor oil monopyroglutamic acid diester monoisostearate, POE-cured castor oil maleic acid and the like); POE-bee wax lanolin derivative (for example, POE-sorbit bee wax and the like); alkanol amide (for example, coconut oil fatty acid diethanol amide, monoethanol amide laurylate, fatty acid isopropanol amide and the like); POE-propylene glycol fatty ester; POE-alkyl amine; POE-fatty acid amide; sucrose fatty acid ester; alkylethoxydimethylamine oxide; trioleyl phosphate and the like are listed.

In order to enhance the user's feeling of using the shampoo and feeling of the quality as a shampoo and suppress the abrasion of hair of scalp at the time when it is washed, it is preferable that it is made so that the surface active agent such as a variety of fatty acid salts and the like which is to be added is in the range from 0.1% by weight to 10% by weight of the total weight of the composition for washing. It is more preferable that it is 8% by weight or more. If it is in this range, the bad influence upon the scalp and hair of scalp is suppressed and fats and fatty oils refilling effects and the like obtained by Hippophae rhamnoides L. fats and fatty oils can be easily secured as well as a suitable foaming is obtained and the user's feeling as a shampoo is obtained.

Moreover, besides fatty acids such as stearic acid, oleic acid and the like, palm oil, jojoba oil, coconut oil or fatty acid salts derived from these fats and fatty oils can be used. Moreover, lecithin, squalan and collagen can be also used.

Moreover, in order to give a suitable viscosity to the composition for washing and enhance the user's feeling of the quality and the user's feeling obtained at the time when it is used, glycerin and the like or a thickening agent such as a thickening polysaccharides and the like can be used. It is preferable that these thickening agents are 10% by weight or less with respect to the total weight of the composition for washing. If it is 10% by weight or less, the shampoo can be easily applied to the whole of the scalp and hair of scalp. It is preferable that it is 5% by weight or less.

Moreover, in the case where the present composition is used as a coloring agent composition for hair of scalp, it is preferable that it contains the component of squid ink as a coloring agent. It is preferable that dried squid ink powder suspended in the above-described water extraction liquid and/or water/alcohol extraction liquid are used. Although it is not particularly limited, it is preferable that for example, squid ink slurry is prepared at the ratio in the range from 100 mL to 1000 mL of the above-described water/alcohol mixture extraction liquid and/or water extraction liquid with respect to 3 g of dried squid ink powder. It is more preferable that it is in the range from 200 mL to 400 mL, and it is still more preferable that it is about 300 mL. Moreover, it is preferable that it is made so that the above-described slurry of squid ink is in the range from 10% by weight to 50% by weight of the whole composition.

Referring to the suspension of squid ink (slurry), it can be manufactured by the procedure in which the dried squid ink powder is immersed in water/alcohol mixed liquid or water with the raw material of the above-described water/alcohol mixed extraction liquid and/or water extraction liquid as well as the above-described extraction liquid can be manufactured. As for the ratio of the extraction liquid, the extraction temperature and the extraction time in this case, the already described ones can be employed as it is. It is preferable that still standing extraction at normal temperature using water/alcohol mixed liquid is used. Specifically, it is still stood in the range from 5°C to 40°C, preferably from 10°C to 37°C, and more preferably from 15°C to 25°C at the light blocking state. In this case, as for the still standing preservation period, it is preferable that it is extracted for a long time period (in the range from about two weeks to about one month, preferably, more than 3 months, and more preferably, it can be made one year or more).

Moreover, the suspension of squid ink may be manufactured by suspending one portion by weight of squid ink powder in the slight amount in the range from about 0.5 portions by weight to about 5 portions by weight of the above-described water extraction liquid and/or water/alcohol extraction liquid or fats and fatty oils of Hippophae rhamnoides L.

As for the amount of squid ink component (dried powder) as a coloring agent component in the above-described hair coloring agent composition, it is different depending upon the dosage form of the coloring agent composition for hair of scalp or the method of utilizing it, however, it can be made in the range from 0.1% by weight to 50% by weight.

In the case where the present composition is used as a coloring agent composition for hair of scalp, it is preferable that gum arabic is contained in the range from 0.5% by weight to 5% by weight of the whole composition. The fixing property of the other active components on the scalp and hair of scalp can be enhanced without damaging the action of fats and fatty oils of Hippophae rhamnoides L. by containing the gum arabic in this range. It is more preferable that it is in the range from 1% by weight to 3% by weight. Moreover, it is preferable that pH of the composition is 6 or less, and it is more preferable that it is in the range from 4.5 to 6.0. To the coloring agent composition, glycerin can be also added. It should be noted that it is preferable that the alcohol concentration of the whole coloring agent for hair of scalp is made 5% by weight or less. Moreover, the coloring agent for hair of scalp containing squid ink component as a coloring agent can be applied to the hair of scalp by a method of lotion and treatment, and the hair of scalp can be gradually colored as well as the hair growing and hair growth effect can be exerted.

### (Method of manufacturing composition for scalp and hair of scalp)

The present composition can be manufactured by appropriately combining and mixing one kind or two kinds or more of a various kinds of components described above. Specifically, the present composition containing the above-described extraction component can be manufactured as an active component. Moreover, the present composition containing the desired effective components of scalp and hair of scalp and components selected from fats and fatty oils of Hippophae rhamnoides L., honey, royal jelly and the like can be manufactured. As for the mixing order of a various kinds and the like are not particularly limited. The present composition is prepared into a dosage form to be targeted by blending and mixing these various kinds of components and so on by the normal method.

Honey and/or royal jelly have been previously mixed with fats and fatty oils of Hippophae rhamnoides L. and the medium composition containing fats and fatty oils of Hippophae rhamnoides L. and honey and/or royal jelly has been previously prepared, and the medium composition and the other composition components are mixed together.

In the preparation of the medium composition, honey and royal jelly have been previously mixed, and subsequently, fats and fatty oils of Hippophae rhamnoides L. are mixed with this mixture. Owing to this, it is easily mixed with fats and fatty oils of Hippophae rhamnoides L. and uniformed, and the separation after the mixture can be suppressed. Moreover, by having done in such a way, a uniform medium composition containing fats and fatty oils of Hippophae rhamnoides L., honey and royal jelly for functioning as a medium of the present composition can be prepared. It should be noted that honey and royal jelly are components of the medium composition from the viewpoint that the flavor peculiar to fats and fatty oils of Hippophae rhamnoides L. can be suppressed as well as these are well dispersed with fats and fatty oils of Hippophae rhamnoides L.

In this medium composition, it is preferable that royal jelly is blended in the range from 3 portions by weight to 10 portions by weight with respect to 1 portion by weight of honey. If it is in this range, a uniform mixture state can be easily obtained as well as both functions of honey and royal jelly can be effectively exerted. It is more preferable that royal jelly is blended in the range from 5 portions by weight to 8 portions by weight with respect to 1 portion by weight of honey.

Moreover, in this medium composition, it is preferable that the total weight of honey and royal jelly are contained in the composition in the range from 3 portions by weight to 15 portions by weight with respect to 1 portion by weight of fats and fatty oils of Hippophae rhamnoides L. Because if it is in this range, the peculiar flavor of fats and fatty oils of Hippophae rhamnoides L. can be suppressed as well as honey and/or royal jelly are well dispersed with fats and fatty oils of Hippophae rhamnoides L. Moreover, if it is less than 3 portions by weight, it tends to be an ununiform mixture state, and if it exceeds over 15 portions by weight, it tends to show the natures of ununiform dispersion, separation in a liquid state and precipitation. It is more preferable that it is in the range from 5 portions by weight to 10 portions by weight.

In a composition for scalp and hair of scalp, it is preferable that the medium composition is 2% by weight of the total weight of it or more. If it is 2% by weight or more, it is possible that it functions as a medium for dispersing and maintaining the other components. It is preferable that it is 5% by weight or more, and it is more preferable that it is 8% by weight or more. Moreover, as for a medium composition, it is preferable that for example, it is 30% by weight or less of the total weight of the composition for scalp and hair of scalp. Because if it is 30% by weight or less, the user's feeling of the excellent quality and user's feeling at the time when it is used over the whole composition can be secured. It can be especially in a washing agent composition for scalp and hair of scalp and the like. It should be noted that in an external preparation composition for scalp and hair of scalp such as a lotion and the like and hair coloring agent composition, it may exceed over 30% by weight and if it is about 90% by weight or less, a suitable feeling at the time when it is used can be obtained.

A composition for scalp and hair of scalp can be prepared by mixing the remaining components with these medium composition. For example, the above-described extraction liquid, the other components and the like can be added to it. It should be noted that the mixture can be performed using a suitable agitating apparatus. The composition for scalp and hair of scalp can be regulated into a preferred value of pH as a composition for scalp and hair of scalp. It is preferable that pH of it is in the range from 4.5 to 6.5. It is more preferable that it is in the range from 4.5 to 5.5. Moreover, as for an oxidation · reduction potential, it is preferable that it is in the range from 90mV to 310 mV.

The present composition described above can grow the hair and bring up the hair while the suppression and prevention of hair loss and epilation are performed and the growth of the hair of scalp is promoted and maintained by containing the above-described extraction composition, by normally functioning the sebaceous membrane of the scalp and cells and improving the scalp environment without chemically damaging the scalp and hair of scalp. Moreover, the present composition can promote the improvement of the scalp environment and the sebaceous membrane of the scalp is appropriately refilled by containing fats and fatty oils of Hippophae rhamnoides L. and when it contains a hair growing and hair growth components such as the above-described extraction components and the like, the effects can be exerted by effectively dispersing and maintaining these components.

Hereinafter, the present invention will be concretely explained by exemplifying Examples, however the present invention is not limited by these Examples. In the following, examples not including an essential components in the amounts as indicated in claim 1 are not part of the invention as claimed.

### Example 1

### (Hair growing and hair growth lotion, not part of the invention as claimed)

### (Example of manufacturing hair growing and hair growth lotion)

1000 mL of water/alcohol mixed solution (35v/v% concentration alcohol (ethanol)) was added to 50g of the raw material which had been prepared at the ratio of 3 portions by weight of Panax japonicus C. A. Meyer with respect to the mixture (total 15 portions by weight) of 10 portions by weight of Reishi Fungus and 5 portions by weight of Coriolus Versicolor and had been finely worked into a spline having the size of 5mm or less, the container was sealed, this container was left as it is in a dark room in which the temperature was controlled at about 15°C, and the natural extraction was performed. After one year passed, the extraction liquid within the sealed container was filtered, and the filtered liquid was made water/alcohol mixture (alcohol concentration 35v/v%) extraction liquid. To 100 mL of this extraction liquid, 900 g of the mixed base of honey and royal jelly and 18 g of gum arabic were added and mixed, to make a hair growing and hair growth lotion. It should be noted that the mixed base of honey and royal jelly was prepared in the following way: 7 portions by weight of royal jelly was added to 2 portions by weight of honey and agitated. The agitation was carried out for 5 minutes at 200 rpm of agitation speed using a stirrer type apparatus, and made it a uniform mixture, and it was made lotion of the present Example. It should be noted that pH of this lotion was 5.5.

### (Evaluation)

The evaluation was performed by making the following subjects use the prepared hair growing and hair growth lotion.

### (Situation of subjects)

### (Subject 1: male, 49 year-old)

Method of using the lotion: The hair growing and hair growth lotion was directly coated on the epilation site with the frequency of once per 7 days. The coating amount was made about 20 mL per each application. This continued for 4 weeks.

Maintenance of scalp and hair of scalp: During the test period, the scalp and the hair of scalp were rinsed once per 2 days with water or warm water. Moreover, during the test period, nothing was coated on the scalp and the hair of scalp except for the lotion of the present Example.

Evaluation: The condition of the scalp and the condition of the hair of scalp located at the epilation site were observed visually by the observer skilled in the art except for the subjects from the initial application to 4 weeks after, and at the same time, the functionality evaluation by the subjects was performed. Moreover, the conditions of the scalp (pH, oil content, water content, tension, moisture, luster) were measured. It should be noted that for pH measurement, a pH measurement device made by Hanna Instruments was used, for oil content and water content, an oil content and water content meter (manufactured by Moritex Co., Ltd., concerning with oil content, reflex method 0 to 15 stage evaluation, concerning with water content, volume method 0 to 15 stage evaluation) was used, and for the tension, moisture and luster, Face Peek manufactured by TANITA, Co., Ltd. was used. The visual observation and functionality evaluation are indicated in Table 1 and the measurements by machines are indicated in Table 2.

**Table 1**

| Item | Before coating | After coating | |
|---|---|---|---|
| | State of scalp and hair of scalp | State of scalp and hair of scalp(1 week after) | State of scalp and hair of scalp (4 weeks after) |
| State of hair of scalp | Epilation state where peripheral scarf skin of parietal region can be admitted | The condition of the hair of scalp on the periphery of the parietal region is not changed | The numerous hairs of scalp just like downy hair grow on the periphery of the parietal region |
| Itching | The itching occurs unless hair washing is performed once per 1 day or 2 days | The itching does not occur. | The itching does not occur. |
| Sticky feeling | The sticky feeling is generated by sebum cutaneum on the next day even if hair washing was performed | The sticky feeling is null if it is rinsed with hot water or water once per 2 or 3 days | The sticky feeling is null if it is rinsed with hot water or water once per 2 or 3 days |
| Hair loss | 20 pieces or more of hairs fall out at the time when it is washed | The condition of hair loss of about 70 to 80% | The state where several pieces of hairs fall out per 1 day |
| State of scalp | The scalp is hard, and parietal region indicates rufous color and the sites of eczema | The scalp becomes softened and The eczema becomes thin | The eczema is not found at the sites, and the color of the scalp approaches to white color |
| State of hair of scalp | The number of hair of scalp from the pit is counted as approximately one piece, and the hair root trace is thin | Sebum cutaneum and dirt of the pit have been reduced | The dirt of the pit has been reduced and the sticky feeing is null |

**Table 2**

| Item | Before coating | 1 week after | 4 weeks after |
|---|---|---|---|
| pH | 5.6 | 4.98 | 5.4 |
| Oil content | 5% | 6% | 9% |
| Water content | 4% | 11% | 5% |
| Tension | 11 | 12 | 12 |
| Moisture | 1 | 1 | 1 |
| Luster | 6 | 6 | 7 |

As indicated in Table 1, one week after the hair growing and hair growth lotion of Example 1 was coated, the itching of the scalp became null, the sticky feeling of the hair of scalp and scalp due to the sebaceous secretion was reduced only by washing the hair with warm water or the like, the amount of hair loss per each day was reduced. Furthermore, 4 weeks after, the peripheral region of the parietal region which was in rufous color became whiter and softened and downy hair began to grow. From these results, according to the present hair growing and hair growth lotion of the present invention, it was understood that it normalizes the sebaceous secretion of the scalp, activates or regenerates the hair root and promotes the hair growing of the hair of scalp.

Moreover, as indicated in Table 2, after the lotion of the present Example was coated, the value of pH of the sebaceous membrane also indicated the ideal value between pH 4.5 to pH 5.5. Moreover, before it is coated, there was a tendency that the water content is short, however, on 1 week after it was coated, an ideal oil content and water content state of the scalp was indicated. As for the tension, moisture and luster, the tension of the scalp (softness) emerged from the time point of 1 week after it was coated. These results sufficiently supported the results of the above-described functionality evaluation and visual observation.

### (Subject 2: male, 49-year-old)

Method of using the lotion: The hair growing and hair growth lotion was directly coated on the epilation sites (mainly, frontal region and parietal region) with the frequency of once per 7 days. The coating amount was made about 10 mL per each application. This has been continued for 6 weeks.

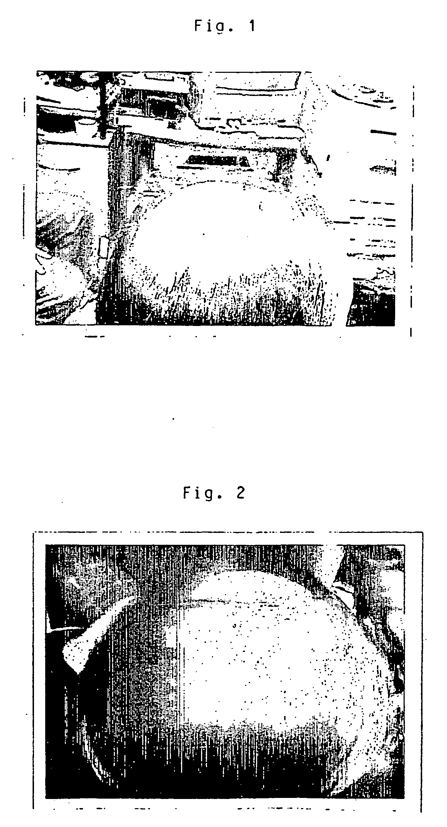

Maintenance of scalp and hair of scalp: During the test period, by setting 7 days as one course by defining the coating day being the first day of the course, and the scalp and the hair of scalp were rinsed on day 3 and day 6 with water or warm water. Moreover, during the test period, nothing was coated on the scalp and the hair of scalp except for the lotion of the present Example. The condition of the epilation site before the start of the test is shown in Fig. 1, and the state of the same site after the test period was completed is shown in Fig. 2.

Results: From the initial application of it to 6 weeks after, there was no sticky feeling and no itching, and the hair of scalp maintained the state of free-flowing. Moreover, as shown in Figs. 1 and 2, the regeneration of the hair of scalp was clearly admitted on the frontal region and the parietal region. On the scalp, there was particularly no eczema, and the scalp was softened.

### (Subject 3: male, 42-year-old)

Method of using the lotion: The hair growing and hair growth lotion was directly coated on the epilation sites (mainly, parietal region) with the frequency of once per 7 days. The coating amount was made about 10 mL per each application. This continued for 9 weeks.

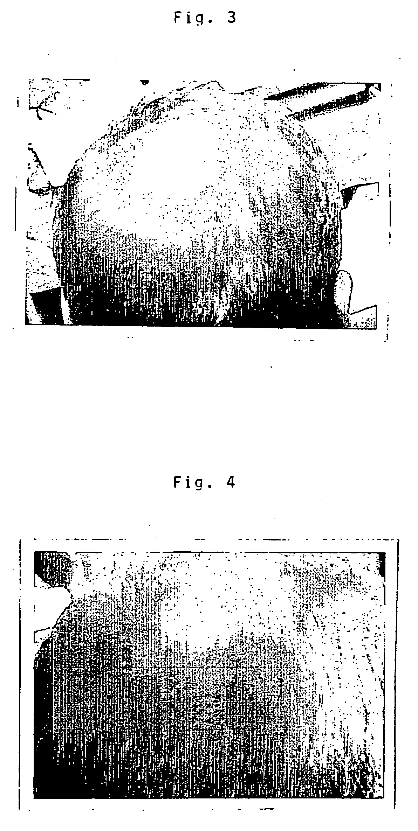

Maintenance of scalp and hair of scalp: During the test period, by setting 7 days as one course by defining the coating day being the first day of the course, and the scalp and the hair of scalp were rinsed on day 3 and day 6 with water or warm water and on day 7, the hair of scalp was washed with a shampoo available in the market. Moreover, during the test period, nothing was coated on the scalp and the hair of scalp except for the lotion of the present Example. The condition of the epilation site before the start of the test is shown in Fig. 3, and the condition of the same site after the test period was completed is shown in Fig. 4.

Results: From the initial application of it to 9 weeks after, there was no sticky feeling and no itching, and the hair of scalp maintained the state of free-flowing. Moreover, as shown in Figs. 3 and 4, the regeneration of the hair of scalp was clearly admitted on the parietal region. On the scalp, there was particularly no eczema, and the scalp was softened.

### Example 2 (not part of the invention as claimed)

### (Hair growing and hair growth shampoo)

### (Example of manufacturing hair growing and hair growth shampoo)

1000 mL of water was added to 18 g of the raw material which had been prepared at the ratio of 3 portions by weight of Panax japonicus C. A. Meyer with respect to the mixture (total 15 portions by weight) of 10 portions by weight of the fruit body of Reishi Fungus and 5 portions by weight of the fruit body of Coriolus Versicolor and had been finely worked into a spline having the size of 5mm or less, it was heated to 95°C by mounting the reflux cooling apparatus and it was maintained for 2 hours. The obtained extraction liquid was filtered and the filtered liquid was made into water extraction liquid. 5 g of fatty acid potassium was added to 10 mL of this water extraction liquid and 10 mL of water/alcohol mixed liquid prepared in Example 1, and it was made into the shampoo of the present Example. It should be noted that pH of the obtained shampoo was 5.9.

### (Evaluation)

The evaluation was performed by making the following subjects use the prepared hair growing and hair growth shampoo.

### (Situation of subjects)

### Gender: male, (44-year-old)

Method of using the shampoo: During the test period, the shampoo of the present Example was used similarly to the conventional method with the frequency of once per 2 days. Moreover, during the test period, nothing was applied on the scalp and the hair of scalp except for the shampoo of the present Example. This has been continued for 2 weeks.

Evaluation: The condition of the scalp and the state of the hair of scalp located at the epilation site were observed visually by the observer skilled in the art except for the subjects from the initial application to 2 weeks after, and at the same time, the functionality evaluation by the subjects was performed. Moreover, the conditions of the scalp (pH, tension, moisture and luster) were measured. It should be noted that the measurement was performed by a method similar to that of Example. The visual observation and functionality evaluation are indicated in Table 3 and the measurements by machines are indicated in Table 4.

**Table 3**

| Item | Before coating | After coating | |
|---|---|---|---|
| | State of scalp and hair of scalp | State of scalp and hair of scalp (1 week after) | State of scalp and hair of scalp (2 weeks after) |
| State of hair of scalp | Hair of scalp nearby the parietal region is thin | The situation of the hair of scalp on the periphery of the parietal region is not changed | The situation of the hair of scalp on the periphery of the parietal region is not changed |
| Itching | The itching occurs unless hair washing is performed once per 2 days | The itching is null | The itching is null |
| Sticky feeling | The sticky feeling is generated by sebum cutaneum on the next day even if hair washing is performed | The free flowing feeling is felt if it is rinsed with hot water or water once per 3 days | The moisture is felt and it continues. The stickiness is not felt. The hair is neither dried nor brittle. |
| Hair loss | 10 pieces or more of hairs fall out at the time when it is washed | The hair loss is reduced to about several pieces of hairs | About several pieces of hairs fall out. |
| State of scalp | The scalp of parietal region indicates reddish color | The reddish color of the parietal region is reduced | The reddish color of the parietal region is null |
| | Sebum cutaneum is attached in the pit and there are many dirts. | Sebum cutaneum and dirts in the pits have been reduced | Sebum cutaneum and dirts in the pits have been reduced |
| State of hair of scalp | There is much oil content of hair of scalp, and there are many dirts. | The dirts of the pit have been reduced | The dirts of the pit have been reduced and the hair of scalp has the moisture content |

**Table 2**

| Item | Before coating | 1 week after | 4 weeks after |
|---|---|---|---|
| pH | 5.77 | 5.38 | 5.48 |
| Tension | 11 | 12 | 12 |
| Moisture | 4 | 5 | 2 |
| Luster | 7 | 8 | 7 |

As indicated in Table 3, after 2 weeks, the number of hair loss has been reduced, the reddish sites located on the scalp was null, the whiteness of sebum cutaneum was recovered, and the tension has emerged. The hair loss of the hair of scalp after the hair was washed was reduced, and the free flowing feeling was felt and it became hair condition in which the hair is well combed. Moreover, as shown in Table 4, after the initiation of using it, the balance of the tension, moisture and luster became excellent as well as the value of pH of the sebaceous membrane indicated the ideal value between pH 4.5 to 5.5.

### Example 3 (not part of the invention as claimed)

### (Coloring agent composition)

### (Manufacturing Example of coloring agent composition)

The squid ink slurry was prepared by adding 1 g of squid ink dried powder to 2 g of water/alcohol mixture extraction liquid prepared in Example 1 and made it as squid ink aqueous solution. Next, 1 g of pure water was added to the mixture in which 4.8 g of royal jelly and 1.2 g of honey were mixed and agitated and dissolved. Furthermore, 10 g of rice paste solution and 1.5 g of gum arabic solution were added to it as a fixing agent, mixed and made into the coloring agent composition. The regulation of the fixing agent was performed by mixing 0.5 g of pure water and 0.5 g of water/alcohol solution with 0.5 g of gum arabic, agitating it and prepared. It should be noted that pH of the composition made in this way was 4.5.

### (Evaluation)

The hair condition after the prepared coloring agent composition was coated (on the hair (white hair portion)) and left as it is and dried was observed and the evaluation was performed.

### (Subject 1: male, 53-year-old)

The white hair became black color similar to he other normal hair. The combing became well. After it was dried, the sticky feeling was not felt, and it was such a natural finishing that the coloring was not admitted. Moreover, since the hair of scalp of the thin portion on the periphery of the parietal region is coated by the coating of the coloring agent composition, there was an effect for covering the thinning of the hair of the scalp. It should be noted that the coloring agent composition is washed by usual hair washing, however, it seemed that the white hair became totally blackened after the hair was washed. Furthermore, on the coated sites, there was no defect such as an eczema on the head and the like, dried and brittle portions were reduced on the whole of the hair including the white hair, and the luster and moisture were increased.

### (Subject 2: male, 44-year-old)

On the white hair located on both sides of the temporal regions, it has been coated, however, it was finished in a natural coloring condition which is the same with the hair of the other sites, there was no sticky feeling after it was dried, and the combing was also well. There was no side-effect such as eczema and the like on the scalp and the hair of the scalp. After the hair was washed using a hair washing agent after 2 days, the white hair became totally blackened.

### (Subject 3: female, 39-year-old)

It was coated on the whole of the hair of the scalp. It was already dyed with a hair dying agent which is available in the market into a dark brown based color, however, the white hair generated was colored with the present agent, after it was colored, it became a natural finishing work as a whole even after it was colored. Moreover, the combing was also well, and the side-effects on the hair of the scalp and the scalp were not admitted. After the present agent was coated, the hair washing was performed 2-3 days later, and the white hair became totally blackened.

### (Subject 4: male, 55-year-old)

It was coated on the whole of the hair of the scalp. After the present agent was coated, the luster and moisture feeling on the hair was recovered, and there was no sticky feeling after it was dried. On the portions where the hair is thin such as the frontal region, parietal region and the like, there was an effect for covering the thinning of the hair by coating the hair of the scalp. There was no defect such as eczema on the head and the like, and the coloring agent was washed with a washing agent.

### Example 4 (not part of the invention as claimed)

### (Hair growing and hair growth lotion)

### (Example of manufacturing hair growing and hair growth lotion)

1000 mL of water/alcohol mixed solution (35v/v% concentration alcohol (ethanol)) was added to 50g of the raw material which had been prepared at the ratio of 3 portions by weight of the root of Panax japonicus C. A. Meyer with respect to the mixture (total 15 portions by weight) of 10 portions by weight of the fruit body of Reishi Fungus and 5 portions by weight of Coriolus Versicolor and had been finely worked into a spline having the size of 5mm or less, the container was sealed, this container was left as it is in a dark room in which the temperature was controlled at about 15°C, and the natural extraction was performed. After one year passed, the extraction liquid within the sealed container was filtered, and the filtered liquid was made water/alcohol mixture liquid (alcohol concentration 35v/v%) extraction liquid.

On the other hand, a hair growing and hair growth lotion of the present Example was made by agitating these at the blending ratio as the followings, respectively, and by making it uniform. It should be noted that the filtered fats and fatty oils of Hippophae rhamnoides L. was used. Moreover, the uniform mixture of honey and royal jelly was prepared by agitating 4 portions by weight of honey in a liquid state and 16 portions by weight of royal jelly frozen and preserved, which had been defrosted at normal temperature and mixing and making it uniform and it was prepared. Moreover, prior to the mixing between water/alcohol mixture extraction liquid and it, the fats and fatty oils of Hippophae rhamnoides L. and honey and royal jelly uniformly mixed matter had been previously mixed and agitated and it was made into a uniform mixture.

### (Blending ratio)

2 g of filtered fats and fatty oils of Hippophae rhamnoides L.
14 g of uniform mixture between 4 portions by weight of honey and 16 portions by weight of royal jelly
1.4 mL of water/alcohol mixture extraction liquid

### (Evaluation)

The evaluation was performed by making the following subjects use the prepared hair growing and hair growth lotion.

### (Situation of subjects)

### (Subject 1: male, 53- year-old)

Method of using the lotion: The hair growing and hair growth lotion was directly coated on the epilation sites with the frequency of once per 7 days. The coating amount was made about 15 mL per each application. Furthermore, subsequently, the hair growing and hair growth lotion was directly coated on the epilation sites with the frequency of once per 14 days. The coating amount was made about 15 mL per each application. This continued for two months.

Maintenance of scalp and hair of scalp : The hair of scalp was rinsed once per 2 or 3 days with water or warm water, and the hair washing was performed with a washing agent which is available in the market once per each week. Evaluation: The condition of the scalp and the condition of the hair of scalp located at the hair loss sites were observed visually by the observer skilled in the art except for the subjects from the initial application to 3 months after, and at the same time, the functionality evaluation by the subjects was performed. The results are indicated in Table 5.

**Table 5**

| Item | Before coating | After coating | |
|---|---|---|---|
| | | 1 month after | 3 months after |
| Hair of scalp | Epilation on the periphery of parietal region | The pit has been observed. Downy hair is regenerated. | The numerous downy hairs are regenerated |
| State of scalp | The scalp of the parietal region is hard, and indicates rufous color | The scalp becomes softened | The scalp becomes softened and whitish |
| Hair loss | Numerous hairs fall out at the time when it is washed | Reduced | Further reduced |
| Sticky feeling | The sticky feeling on the hair of scalp | Reduced | Reduced |
| Itching | Some | Reduced | None |

As indicated in Table 5, 4 weeks after the hair growing and hair growth lotion of Example 4 was coated, the condition of the scalp and the hair of the scalp was improved. Furthermore, after 8 weeks passed following the coating, the regeneration of the downy hair was admitted on the depilation portion, and the condition of the scalp and the hair of scalp has been further improved. From these results, according to the present hair growing and hair growth lotion, it was understood that it normalizes the sebaceous secretion of the scalp, activates or regenerates the hair root and promotes the hair growing of the hair of scalp.

### (Subject 2: male, 43- year-old)

Method of using the lotion: The hair growing and hair growth lotion was directly coated on the epilation sites with the frequency of once per 7 days. The coating amount was made about 15 mL per each application. This has been continued for two months. Furthermore, subsequently, the hair growing and hair growth lotion was directly coated on the epilation sites with the frequency of once per 14 days. The coating amount was made about 15 mL per each application. This continued for one month.

Maintenance of scalp and hair of scalp: The hair of scalp was rinsed once per 2 or 3 days with warm water, and the hair washing was performed with a washing agent which is available in the market once per each week.

Evaluation: The condition of the scalp and the condition of the hair of scalp located at the epilation sites have been observed visually by the observer skilled in the art except for the subjects from the initial application to 12 weeks after, and at the same time, the functionality evaluation by the subjects was performed. The results are indicated in Table 6.

**Table 6**

| Item | Before coating | After coating | |
|---|---|---|---|
| | | 1 month after | 3 months after |
| Hair of scalp | On the frontal region and the parietal region, 80 - 90% were depilated. | Downy hair is regenerated on the frontal region. | The numerous downy hairs are regenerated on the frontal region and the parietal region |
| State of scalp | Rufous color - brown color | The scalp becomes softened | The scalp becomes softened and whitish. |
| Hair loss | The Numerous hairs fall out at the time when it is combed | Reduced | Further reduced |
| Sticky feeling | The sticky feeling on the hair of scalp | Reduced | None |
| Itching | Rather some | Reduced | None |

Results: After one month passed from the initial application, the conditions of the scalp and the hair of the scalp were improved. After 3 months passed, the downy hair was also regenerated, and the conditions of the scalp and the hair of the scalp were further improved. From these results, according to the present hair growing and hair growth lotion, it was understood that it normalizes the sebaceous secretion of the scalp, activates or regenerates the hair root and promotes the hair growing of the hair of scalp.

### (Subject 3: male, 49- year-old)

Method of using the lotion: The hair growing and hair growth lotion was directly coated on the epilation sites (mainly on the parietal region) with the frequency of once per 7 days. The coating amount was made about 15 mL per each application. This has been continued for one month.

Maintenance of scalp and hair of scalp: The hair of scalp was rinsed once per 2 days with water or warm water, and the hair washing was performed with a washing agent which is available in the market once per each week.

Evaluation: The condition of the scalp and the condition of the hair of scalp located at the epilation sites have been observed visually by the observer skilled in the art except for the subjects from the initial application to one month after, and at the same time, the functionality evaluation by the subjects was performed. The results are indicated in Table 7. Moreover, the conditions of the scalp (pH, oil content, water content, tension, moisture, luster) were measured. It should be noted that for pH measurement, a pH measurement device made by Hanna Instruments was used, for oil content and water content, an oil content and water content meter (manufactured by Moritex Co., Ltd., concerning with oil content, reflex method 0 to 15 stage evaluation, concerning with water content, volume method 0 to 15 stage evaluation) was used, and for the tension, moisture and luster, Face Peek manufactured by TANITA, Co., Ltd. was used. The measurements by machines are indicated in Table 8.

**Table 7**

| Item | Before coating | After coating | |
|---|---|---|---|
| | | 1 month after | 3 months after |
| Hair of scalp | The hairs were almost completely depilated on the parietal region | No change | The numerous downy hairs are regenerated on the parietal region |
| State of scalp | The parietal region is hard, and rufous color - brown color | The scalp becomes softened | The scalp becomes softened and whitish. |
| Hair loss | The Numerous hairs fall out at the time when it is combed | Reduced | Further reduced |
| Sticky feeling | The sticky feeling on the hair of scalp | Reduced | None |
| Itching | Some | Reduced | None |

**Table 8**

| Item | Before coating | 1 week after | 4 weeks after |
|---|---|---|---|
| pH | 5.6 | 5.5 | 5.4 |
| Oil content | 5% | 7% | 7% |
| Water content | 4% | 11% | 6% |
| Tension | 11 | 11 | 12 |
| Moisture | 1 | 1 | 1 |
| Luster | 6 | 6 | 8 |

Results: After one week passed from the initial application, the conditions of the scalp and the hair of the scalp were improved. After 4 weeks passed, the downy hair was also regenerated as well as the conditions of the scalp and the hair of the scalp were further improved. Moreover, the measurement results by the machines have sufficiently supported the improvement of the conditions of scalp. From these results, according to the present hair growing and hair growth lotion, it was understood that it normalizes the sebaceous secretion of the scalp, activates or regenerates the hair root and promotes the hair growing of the hair of scalp.

### Example 5 (not part of the invention as claimed)

### (Hair growing and hair growth shampoo)

### (Example of manufacturing hair growing and hair growth shampoo)

1000 mL of water was added to 18 g of the raw material which had been prepared at the ratio of 3 portions by weight of the root of Panax japonicus C. A. Meyer with respect to the mixture (total 15 portions by weight) of 10 portions by weight of the fruit body of Reishi Fungus and 5 portions by weight of the fruit body of Coriolus Versicolor had been finely worked into a spline having the size of 5mm or less, it was heated to 95°C by mounting the reflux cooling apparatus and it was maintained for 2 hours. The obtained extraction liquid was filtered and the filtered liquid was made into water extraction liquid. 1.5 g of fatty acid potassium and 10 mL of pure water were added to 10 mL of water extraction liquid, 3 g of the medium prepared in Example 4, and about 5.5 mL of water/alcohol mixture extraction liquid prepared in Example 4, uniformly agitated and mixed and it was made into the shampoo of the present Example. It should be noted that pH of the obtained shampoo was 5.9.

### (Evaluation)

The evaluation was performed by making the following subjects use the prepared hair growing and hair growth shampoo.

### (Subject 1: male, 53-year-old)

Method of using the shampoo: Before a shampoo according to the present invention is used, after the hair of scalp was washed and wetted with warm water, the present shampoo was coated over the whole hair of scalp and washed by hands. The hair washing time period was about 3 minutes, and subsequently, the scalp and the hair of the scalp were rinsed with warm water and the water content attached on was wiped with a towel. During the test period, the hair washing was performed once per 1 day or 2 days using the shampoo of the present Example. Moreover, during the test period, nothing was applied to the scalp and the hair of scalp except for the shampoo of the present Example. This has been continued for 4 weeks.

Evaluation: The condition of the scalp and the condition of the hair of scalp located at the epilation site have been observed visually by the observer skilled in the art except for the subjects from the initial application to 4 weeks after, and at the same time, the functionality evaluation by the subjects was performed. Moreover, the conditions of the scalp (pH, tension, moisture and luster) were measured. It should be noted that the measurement was performed by a method similar to that of Example 1. The visual observation and functionality evaluation are indicated in Table 9 and the measurements by machines are indicated in Table 10.

**Table 9**

| Item | Before its use | After its use |
|---|---|---|
| | | 4 weeks after |
| Hair of scalp | In the case of shampoo available in the market, there are dried and brittle feelings after it is washed | No dried and brittle feelings after it is washed. The numerous downy hairs are regenerated on the frontal region of suprafront region and the parietal region |
| State of scalp | The parietal region is hard, and rufous color - brown color | The scalp becomes softened |
| Hair loss | The Numerous hairs fall out at the time when it is washed | Reduced |
| Sticky feeling | Not known because of the use of hair styling products | None |
| Itching | Some | None |

**Table 10**

| Item | Before its use | After its use |
|---|---|---|
| | | 4 weeks after |
| pH | 6 | 5.5 |
| Oil content | 4% | 4% |
| Water content | 1% | 4% |
| Tension | 12 | 12 |
| Moisture | 1 | 1 |
| Luster | 8 | 12 |

As indicated in Table 9, After 4 weeks passed from the initial application, the conditions of the scalp and the hair of the scalp were improved. Moreover, the downy hairs were regenerated on the parietal region and the frontal region. Moreover, as indicated in Table 10, after 4 weeks passed, the balance of the tension, moisture and luster became excellent as well as the value of pH of the sebaceous membrane also indicated the ideal value between pH 4.5 to pH 5.5. Moreover, the measurement results by the machines have sufficiently supported the improvement of the conditions of scalp. From these results, according to the present hair growing and hair growth shampoo, it was understood that it normalizes the sebaceous secretion of the scalp, activates or regenerates the hair root and promotes the hair growing of the hair of scalp.

### (Subject 2: male, 55-year-old)

Method of using the shampoo: Before a shampoo according to the present invention was used, after the hair of scalp was washed and wetted with warm water, the present shampoo was coated over the whole hair of scalp and washed by hands. The hair washing time period was about 3 minutes, and subsequently, the scalp and the hair of the scalp were rinsed with warm water and the water content attached on them was wiped with a towel. During the test period, the hair washing was performed similarly to the conventional method once per 1 day or 2 days using the shampoo of the present Example. Moreover, during the test period, nothing was applied to the scalp and the hair of scalp except for the shampoo of the present Example. This has been continued for 4 weeks.

Evaluation: The condition of the scalp and the condition of the hair of scalp located at the epilation site have been observed visually by the observer skilled in the art except for the subjects from the initial application to 4 weeks after, and at the same time, the functionality evaluation by the subjects was performed. Moreover, the conditions of the scalp (pH, tension, moisture and luster) were measured similar to the case of the subject 1. The visual observation and functionality evaluation are indicated in Table 11 and the measurements by machines are indicated in Table 12.

**Table 11**

| Item | Before its use | After its use |
|---|---|---|
| | | 4 weeks after |
| Hair of scalp | In the case of shampoo available in the market, there are dried and brittle feelings after it is washed | No dried and brittle feelings after it is washed. The numerous downy hairs are regenerated on the parietal region |
| State of scalp | Hard and rufous color | The scalp becomes softened |
| Hair loss | The Numerous hairs fall out at the time when it is washed | Reduced |
| Sticky feeling | The sticky feeling on the whole hair of scalp | Reduced |
| Itching | Some dandruff | None of itching, dandruff |

**Table 12**

| Item | Before its use | After its use |
|---|---|---|
| | | 4 weeks after |
| pH | 5.71 | 5.65 |
| Oil content | 14% | 12% |
| Water content | 0% | 3% |
| Tension | 11 | 13 |
| Moisture | 1 | 1 |
| Luster | 5 | 9 |

As indicated in Table 11, after 4 weeks passed from the initial application, the conditions of the scalp and the hair of the scalp were improved. Moreover, the downy hairs were regenerated on the parietal region and the frontal region. Moreover, as indicated in Table 12, after 4 weeks passed, the tension and luster of the scalp were increased as well as the value of pH of the sebaceous membrane also indicated the ideal value. Moreover, the measurement results by the machines have sufficiently supported the improvement of the conditions of scalp. From these results, according to the present hair growing and hair growth lotion, it was understood that it normalizes the sebaceous secretion of the scalp, activates or regenerates the hair root and promotes the hair growing of the hair of scalp.

### Example 6 (not part of the invention as claimed)

### (Coloring agent composition)

### (Manufacturing Example of coloring agent composition)

1 g of fats and fatty oils of Hippophae rhamnoides L. was added to 1 g of squid ink dried powder, agitated and mixed. Next, 2 g of honey was added to 8 g of royal jelly, agitated and mixed. 1 g of water/alcohol mixture extraction liquid prepared in Example 4 was added to 2 g of the former mixture and 7 g of the latter mixture and made into the coloring agent composition.

### (Evaluation)

The hair condition after the coloring agent composition prepared was coated on the dried hair (white hair portion) of the subject and left as it is and dried was observed and the evaluation was performed by observing the condition after washing the hair.

### (Subject 1: male, 50-year-old)

The white hair became black color similar to the normal hair by coating the present composition. The combing became well. It was a natural finishing as the effect after the coloring was performed and there was no sense of discomfort. The coloring agent composition is washed by usual hair washing, however, it seemed that the white hair became totally blackened after the hair was washed. Furthermore, there was no defect such as eczema on the scalp and the like, and the luster and moisture were increased. The pH of the scalp coated in this way indicated 5.12.

### (Subject 2 : male, 50-year-old)

Focusing on the white hair located on the parietal region and both sides of the temporal regions, it has been coated over the whole region. It was finished in a natural black coloring condition. The combing was also well. There was no side-effect such as eczema and the like on the scalp and the hair of the scalp. After the hair was washed using a hair washing agent after 2 days, the white hair became totally blackened. The pH of the scalp indicated 5.5 and the most suitable pH for the scalp and the hair of the scalp was indicated.

### (Subject 4: male, 45-year-old)

It was coated on the whole of the hair of the scalp since there were white hairs all over the hair of scalp. After it was colored, the luster and moisture feeling on the hair was recovered, and the combing was also well. The side-effects on the hair of the scalp and the scalp were not admitted. The pH of the scalp indicated 4.98 and the most suitable pH for the scalp and the hair of the scalp was indicated.

### (Subject 5: male, 51-year-old)

It was coated on the whole of the hair of the scalp. After the present agent was coated, the luster and moisture feeling on the hair was recovered. The coming was also well and there was no defect such as eczema on the hair of the scalp and the scalp. The pH of the scalp indicated 5.06 and the most suitable pH for the scalp and the hair of the scalp was indicated.

### (Subject 6: male, 40-year-old)

It was coated on the whole of the hair of the scalp since the numerous white hairs covers the whole hair of scalp. After the present agent was coated, the luster and moisture feeling on the hair was recovered. The coming was also well and there was no defect such as eczema on the hair of the scalp and the scalp. The pH of the scalp indicated 5.0 and the most suitable pH for the scalp and the hair of the scalp was indicated.

From the description described above, it was understood that the present coloring composition has the effect of temporary coloring of the hair of scalp and also has a continuous coloring effect. Moreover, the present coloring agent composition can make the scalp and the hair of the scalp healthy without damaging the scalp and the hair of the scalp.

### Example 7 (according to the invention)

### (Manufacturing Example 1 of composition for washing)

### (1) Preparation of medium composition

The medium composition containing 7 g of royal jelly, 1.8 g of honey and 1.2 g of fats and fatty oils of Hippophae rhamnoides L. was prepared. First, honey was added to royal jelly, and after uniformly mixed, further, the fats and fatty oils of Hippophae rhamnoides L. was added, mixed and made into the uniform medium composition (10 g).

### (2) Preparation of extraction liquid

10 g of the fruit body of Reishi Fungus and 5 g of the fruit body of Coriolus Versicolor and 3 g of red ginseng were added to 1 L of water and extracted using hot water for 2 hours to prepare water extraction liquid. Moreover, 10 g of the fruit body of Reishi Fungus and 5 g of the fruit body of Coriolus Versicolor and 3 g of red ginseng were added to 1 L of 35% ethanol solution and extracted for 24 hours while it was refluxed at 75°C to prepare water/alcohol mixture extraction liquid.

### (3) Preparation of composition for washing

20 g of water extraction liquid and 10 g of water/alcohol mixture extraction liquid were added to 10 g of the medium composition, and further, 46.5 g of water was added and mixed to prepare the stock solution of the composition. 10 g of fatty acid potassium, 2 g of glycerin and 1.5 g of the thickening component were added to the stock solution, mixed, and made into a composition for washing. In this composition for washing the following composition is involved:
Royal jelly : (7% by weight)
Honey : (1.8% by weight)
Hippophae rhamnoides L.: (1.25 by weight)
Hot water extraction solution: 10 g of Reishi Fungus + 5 g of Coriolus versicolor + 3 g of red ginseng/L : (20% by weight)
Alcohol extraction solution: 10 g of Reishi Fungus + 5 g of Coriolus versicolor + 3 g of red ginseng/L : (10% by weight)
Fatty acid potassium : (10% by weight)
Water: (46.5% by weight)
Glycerin : (2% by weight) and
Thickening component : (1.5% by weight).

### Example 8 (not part of the invention as claimed)

### (Manufacturing Example 2 of composition for washing)

### (1) Preparation of medium composition

The medium composition containing 4 g of royal jelly, 0.5 g of honey and 0.5 g of fats and fatty oils of Hippophae rhamnoides L. was prepared. First, honey was added to royal jelly, and after uniformly mixed, further, the fats and fatty oils of Hippophae rhamnoides L. was added, mixed and made into the uniform medium composition (5 g).

### (2) Preparation of extraction liquid

10 g of the fruit body of Reishi Fungus and 5 g of the fruit body of Coriolus Versicolor and 3 g of red ginseng were added to 1 L of water and extracted using hot water for 2 hours to prepare water extraction liquid. Moreover, 10 g of the fruit body of Reishi Fungus and 5 g of the fruit body of Coriolus Versicolor and 3 g of red ginseng were added to 1 L of 35% ethanol solution and extracted for 24 hours while it was refluxed at 75°C to prepare water/alcohol mixture extraction liquid.

### (3) Preparation of composition for washing

33.3 g of water extraction liquid and 16.7 g of water/alcohol mixture extraction liquid were added to 10 g of the medium composition, and further, 29.5 g-35.5 g of water was added and mixed to prepare the stock solution of the composition. 6 g-12 g of the respective surface active agent of fatty acid sodium, fatty acid potassium, sodium laureth sulfate and cocamide propyl betaine were added so that the combination of this stock solution and water additional weight becomes 41.5 g, and further, 2 g of glycerin was added, and Total 14 kinds of composition for washing were prepared. It should be noted that the surface active agents of these 14 kinds and their contents were as indicated in Table 13. In these compositions for washing, the following composition is involved:
Royal jelly: (4% by weight)
Honey : (0.5% by weight)
Hippophae rhamnoides L.: (0.5% by weight)
Hot water extraction solution: 10 g of Reishi Fungus + 5 g of Coriolus versicolor + 3 g of red ginseng/L : (33.3% by weight)
Alcohol extraction solution: 10 g of Reishi Fungus + 5 g of Coriolus versicolor + 3 g of red ginseng/L : (16.7% by weight)
Various kinds of surface active agents : (6 - 12% by weight)
Water : (46.5% by weight)
Glycerin : (2% by weight) and
Thickening component : (1.5% by weight).

**Table 13**

| Surface active agent | | Foaming height (mm) | | |
|---|---|---|---|---|
| Content | Kinds | immediately after frothing | after 30 minutes | after 24 hours |
| 6% by weight | Fatty acid Na | 28 | 20 | 2 |
| | Fatty acid K | 20 | 20 | 0 |
| | Laureth sulfate | 35 | 25 | 1 |
| | cocamide based | 50 | 38 | 3 |
| 8% by weight | Fatty acid Na | 38 | 31 | 5 |
| | Fatty acid K | 30 | 25 | 0 |
| | Laureth sulfate | 40 | 32 | 1 |
| | cocamide based | 50 | 40 | 3 |
| 10% by weight | Fatty acid Na | 48 | 30 | 5 |
| | Fatty acid K | 50 | 40 | 0 |
| | Laureth sulfate | 48 | 34 | 1 |
| | Cocamide based | 55 | 41 | 3 |
| 12% by weight | Laureth sulfate | 52 | 36 | 1 |
| | cocamide based | 65 | 35 | 2 |

### (4) Evaluation of foaming performance

With respect to 100 mL of various kinds of compositions for washing prepared in (3), the total 3 times of agitation operations (handy agitation machine, 10 seconds, respectively, the same strength) of immediately after the preparation, 30 minutes after from it, and 24 hours from it were performed and the frothing was performed, and from the time immediately after the frothing, the foaming height (mm) after the predetermined passing time period were measured. The whole results are indicated in Table 13.

As indicated in Table 13, on any one of surface active agents, an excellent user's feeing at the time when it is used and foaming property were obtained in the range from 6% by weight to 12% by weight. Among these, an excellent and stable frothing property was provided in the range from 8% by weight to 10% by weight, the user's feeling at the time when it is used was also still more excellent. On the other hand, in the case of 6% by weight, the tendency that frothing is rather inferior in frothing emerges, and in the case of 12% by weight, the tendency of excessive frothing was observed.

The present invention is based on the priority claim of Japanese Patent Application No. 2004-152083 Gazette which was filed on May 21, 2004 and Japanese Patent Application No. 2005-037942 Gazette which was filed on February 15, 2005, and the entire contents of it have been incorporated.

## Claims

1. A composition for scalp and hair of scalp,
the composition containing extraction component of fungi belonging to basidiomycetes, extraction component of the root of a plant of Araliaceae, honey and/or royal jelly, wherein the composition further contains fats and fatty oils of Hippophae rhamnoides L. whose raw material is fruit and/or seed of Elaeagnaceae Hippophae rhamnoides L. plant,
wherein, as the total amount, honey and/or royal jelly are contained in the composition in the range from 3 portions by weight to 15 portions by weight with respect to 1 portion by weight of fats and fatty oils of Hippophae rhamnoides L., and
wherein the total amount of honey and/or royal jelly in the composition is in the range from 5 % by weight to 90 % by weight.

2. The composition as claimed in Claim 1, in which fungi belonging to the basidiomycetes contain fungi belonging to Aphyllophorales Ganodermataceae Ganoderma lucidum and/or fungi belonging to Aphyllophorales Polyporaceae Coriolus.

3. The composition as claimed in Claim 2, in which fungus belonging to the Ganoderma lucidum is Reishi Fungus.

4. The composition as claimed in Claim 2 or 3, in which fungus belonging to the Coriolus is Coriolus Versicolor.

5. The composition as claimed in any one of Claims 1 to 4, in which the plant of Araliaceae is Panax japonicus C. A. Meyer.

6. The composition as claimed in any one of Claims 1 to 5, in which extraction component of fungus belonging to the basidiomycetes and extraction component of the root of the plant of Araliaceae are water extraction component and/or water/alcohol mixture extraction component, respectively.

7. The composition as claimed in Claim 6, in which water extraction component of fungus belonging to the basidiomycetes contains the component in the extraction liquid obtained by immersing the cultured matter of fungus belonging to the basidiomycetes, by blocking the light and by still standing and preserving it in the range from 5°C to 40°C.

8. The composition as claimed in Claim 6 or 7, in which extraction component of the root of the Araliaceae contains the component in the extraction liquid obtained by immersing the root of the Araliaceae, by blocking the light and by still standing and preserving it in the range from 5°C to 40°C.

9. The composition as claimed in any one of Claims 1 to 8, in which
the composition has raw material composition in the range from 0.2 portions by weight to 20 portions by weight of the root of a plant of the Araliaceae with respect to 20 portions by weight of fungi belonging to the basidiomycetes.

10. The composition as claimed in any one of Claims 1 to 9, in which the composition further contains squid ink component.

## Patentansprüche

1. Zusammensetzung für Kopfhaut und Kopfhaar,
wobei die Zusammensetzung eine Extraktionskomponente von Pilzen, die zu Basidiomyceten gehören, eine Extraktionskomponente der Wurzel einer Araliaceae-Pflanze, Honig und/oder Gelee Royale enthält, wobei die Zusammensetzung ferner Fette und fette Öle von Hippophae rhamnoides L. enthält, deren Ausgangsmaterial die Frucht und/oder der Samen der Elaeagnaceae Hippophae rhamnoides L.-Pflanze ist,
wobei, als Gesamtmenge, Honig und/oder Gelée Royale in der Zusammensetzung im Bereich von 3 Gewichtsteilen bis 15 Gewichtsteilen bezogen auf 1 Gewichtsteil von Fetten und fetten Ölen von Hippophae rhamnoides L. enthalten ist oder sind, und
wobei die Gesamtmenge von Honig und/oder Gelée Royale in der Zusammensetzung im Bereich von 5 Gewichtsprozent bis 90 Gewichtsprozent liegt.

2. Zusammensetzung nach Anspruch 1, bei der die Pilze, die zu den Basidiomyceten gehören, Pilze enthalten, die zu Aphyllophorales Ganodermataceae Ganoderma lucidum gehören, und/oder Pilze enthalten, die zu Aphyllophorales Polyporaceae Coriolus gehören.

3. Zusammensetzung nach Anspruch 2, bei welcher der Pilz, der zu dem Ganoderma lucidum gehört, ein Reishi-Pilz ist.

4. Zusammensetzung nach Anspruch 2 oder 3, bei welcher der Pilz, der zu dem Coriolus gehört, Coriolus Versicolor ist.

5. Zusammensetzung nach einem der Ansprüche 1 bis 4, bei der die Araliaceae-Pflanze Panax japonicus C. A. Meyer ist.

6. Zusammensetzung nach einem der Ansprüche 1 bis 5, bei der die Extraktionskomponente des Pilzes, der zu den Basidiomyceten gehört, und die Extraktionskomponente der Wurzel der Araliaceae-Pflanze jeweils eine Wasserextraktionskomponente und/oder eine Wasser/Alkohol-Gemisch-Extraktionskomponente sind.

7. Zusammensetzung nach Anspruch 6, bei der die Wasserextraktionskomponente des Pilzes, der zu den Basidiomyceten gehört, die Komponente in der Extraktionsflüssigkeit enthält, die durch Eintauchen des gezüchteten Materials des Pilzes, der zu den Basidiomyceten gehört, unter Lichtausschluss und durch ruhiges Stehenlassen und Halten derselben im Bereich von 5 °C bis 40 °C erhalten worden ist.

8. Zusammensetzung nach Anspruch 6 oder 7, bei der die Extraktionskomponente der Araliaceae-Wurzel die Komponente in der Extraktionsflüssigkeit enthält, die durch Eintauchen der Araliaceae-Wurzel unter Lichtausschluss und durch ruhiges Stehenlassen und Halten derselben im Bereich von 5 °C bis 40 °C erhalten worden ist.

9. Zusammensetzung nach einem der Ansprüche 1 bis 8, wobei
die Zusammensetzung eine Ausgangsmaterialzusammensetzung im Bereich von 0,2 Gewichtsteilen bis 20 Gewichtsteilen der Wurzel einer Araliaceae-Pflanze bezogen auf 20 Gewichtsteile der Pilze, die zu den Basidiomyceten gehören, aufweist.

10. Zusammensetzung nach einem der Ansprüche 1 bis 9, wobei die Zusammensetzung ferner eine Tintenfischtintenkomponente enthält.

## Revendications

1. Composition pour le cuir chevelu et pour les cheveux, la composition contenant un composant d'extraction tiré
de champignons appartenant à la classe des basidiomycètes, un composant d'extraction tiré d'une racine de plante de la famille Araliaceae, du miel et/ou de la gelée royale, dans laquelle la composition contient en outre des graisses et des huiles grasses tirées de l'espèce Hippophae rhamnoides L., dont la matière brute est le fruit et/ou la graine de la plante Elaeagnaceae Hippophae rhamnoides L.,
dans laquelle, en quantité totale, le miel et/ou la gelée royale sont contenus dans la composition dans la plage de 3 parties en poids à 15 parties en poids par rapport à 1 partie en poids de graisses et d'huiles grasses de l'espèce Hippophae rhamnoides L., et
dans laquelle la quantité totale de miel et/ou de gelée royale dans la composition se situe dans la plage de 5 % en poids à 90 % en poids.

2. Composition selon la revendication 1, dans laquelle les champignons appartenant à la classe des basidiomycètes contiennent des champignons appartenant à l'espèce Aphyllophorales Ganodermataceae Ganoderma lucidum et/ou des champignons appartenant à l'espèce Aphyllophorales Polyporaceae Coriolus.

3. Composition selon la revendication 2, dans laquelle le champignon appartenant à l'espèce Ganoderma lucidum est le Reishi Fungus.

4. Composition selon la revendication 2 ou 3, dans laquelle le champignon appartenant au genre Coriolus est l'espèce Coriolus Versicolor.

5. Composition selon l'une quelconque des revendications 1 à 4, dans laquelle la plante de la famille Araliaceae est l'espèce Panax japonicus C. A. Meyer.

6. Composition selon l'une quelconque des revendications 1 à 5, dans laquelle le composant d'extraction du champignon appartenant à la classe des basidiomycètes et le composant d'extraction de la racine de plante de la famille Araliaceae sont des composants d'extraction à l'eau et/ou des composants d'extraction avec un mélange eau/alcool, respectivement.

7. Composition selon la revendication 6, dans laquelle le composant d'extraction à l'eau du champignon appartenant à la classe des basidiomycètes contient le composant présent dans le liquide d'extraction obtenu en immergeant la matière de culture du champignon appartenant à la classe des basidiomycètes, en bloquant la lumière tout en la maintenant et en l'entretenant encore dans la plage de 5 °C à 40 °C.

8. Composition selon la revendication 6 ou 7, dans laquelle le composant d'extraction de la racine de plante de la famille Araliaceae contient le composant présent dans le liquide d'extraction obtenu en immergeant la racine d'une plante de la famille Araliaceae, en bloquant la lumière tout en la maintenant et en l'entretenant encore dans la plage de 5 °C à 40 °C.

9. Composition selon l'une quelconque des revendications 1 à 8, dans laquelle
la composition présente une composition de matière brute dans la plage de 0,2 partie en poids à 20 parties en poids de la racine d'une plante de la famille Araliaceae par rapport à 20 parties en poids des champignons appartenant à la classe des basidiomycètes.

10. Composition selon l'une quelconque des revendications 1 à 9, dans laquelle la composition contient en outre un composant d'encre de sèche.
